(19) Europäisches Patentamt
European Patent Office
Office européen des brevets

(11) EP 4 283 283 A1

(12) **EUROPEAN PATENT APPLICATION**
published in accordance with Art. 153(4) EPC

(43) Date of publication:
**29.11.2023 Bulletin 2023/48**

(21) Application number: **21921285.9**

(22) Date of filing: **16.12.2021**

(51) International Patent Classification (IPC):
**G01N 22/04** (2006.01)  **G01N 22/00** (2006.01)

(52) Cooperative Patent Classification (CPC):
**G01N 22/00; G01N 22/04**

(86) International application number:
**PCT/JP2021/046431**

(87) International publication number:
**WO 2022/158183 (28.07.2022 Gazette 2022/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.01.2021 JP 2021006339**

(71) Applicant: **Sony Group Corporation**
**Tokyo 108-0075 (JP)**

(72) Inventors:
• IIDA Sachio
  **Tokyo 108-0075 (JP)**
• YAMADA Atsushi
  **Tokyo 108-0075 (JP)**
• ICHIHARA Takuya
  **Atsugi-shi, Kanagawa 243-0014 (JP)**
• OISHI Takahiro
  **Tokyo 108-0075 (JP)**
• HIROI Toshiyuki
  **Tokyo 108-0075 (JP)**

(74) Representative: **D Young & Co LLP**
**120 Holborn**
**London EC1N 2DY (GB)**

(54) **MEASURING DEVICE AND MEASURING METHOD**

(57)     [Problem] Provided are a measurement device and a measurement method that enable calibration in the event of error variations.

[Solution] A measurement device configured to measure an amount of moisture contained in a medium, the measurement device including: a first probe in which a first cable electrically connectable to a first connection cable is embedded; a second probe in which a second cable electrically connectable to a second connection cable is embedded; and a standard that is fixed at a predetermined positional relationship with the first probe and the second probe even during the measurement, is electrically connectable to the first connection cable and the second connection cable when the measurement is not conducted, and is used for calibration of the measurement.

Fig. 1

**Description**

[Technical Field]

The present disclosure relates to a measurement device and a measurement method.

[Background Art]

Measurement devices for measuring amounts of moisture in media such as soil are widely used in the fields of agriculture and environmental research or the like. For example, a proposed measurement device measures an amount of moisture from a propagation delay time for the propagation of electromagnetic waves through a medium between a pair of probes. This measurement device connects the pair of probes to a transmitter and a receiver via a cable, transmits an electric signal from the transmitter to the receiver, and determines a delay time from the transmission to the reception. Furthermore, the measurement device has stored, as an error of a fixed value, a transmission time for transmitting the electric signal through the cable and subtracts the error from the determined delay time, thereby determining the propagation delay time for the propagation of electromagnetic waves through the medium.

Typically, for such measurement devices, errors are measured to be corrected at the factory shipments, and calibration factors are stored. Thus, the amounts of moisture are measured according to measurement data calibrated by the calibration factors.

[Citation List]

[Patent Literature]

[PTL 1]
WO 2018/221051
[PTL 2]
Japanese Patent Application No. H08-300197

[Summary]

[Technical Problem]

However, the length of the cable may be changed by thermal expansion and cause variations of errors. Accordingly, in the sensor device that stores the error as a fixed value, the accuracy of measurement of the amount of moisture may decrease when the cable thermally expands.

Hence, the present disclosure proposes a measurement device and a measurement method that enable calibration in the event of error variations.

[Solution to Problem]

In order to solve the above problem, the present disclosure provides a measurement device configured to measure an amount of moisture contained in a medium, the measurement device including:

a first probe in which a first cable electrically connectable to a first connection cable is embedded;
a second probe in which a second cable electrically connectable to a second connection cable is embedded; and
a standard that is fixed at a predetermined positional relationship with the first probe and the second probe even during the measurement, is electrically connectable to the first connection cable and the second connection cable when the measurement is not conducted, and is used for calibration of the measurement.

The measurement device may further include a switching unit that switches connection between the first connection cable and the first probe or the standard and connection between the second connection cable and the second probe or the standard.

The switching unit may include:

a first switching unit that switches the connection between the first connection cable and the first probe or the standard; and
a second switching unit that switches the connection between the second connection cable and the second probe or the standard.

The standard may include an open standard, a short standard, and a reflection-free termination and further include a first terminal for connection to the open standard, a second terminal for connection to the short standard, a third terminal for connection to the reflection-free termination, and one fourth terminal and the other fourth terminal for direct connection to the first connection cable and the second connection cable.

The first switching unit may switch connection between the first connection cable and one of the first probe, the first to third terminals, and the one fourth terminal, and the second switching unit may switch connection between the second connection cable and the second probe or the other fourth terminal.

The first probe, the second probe, the standard, the first switching unit, and the second switching unit may be configured in the same housing.

The measurement device may further include a coefficient calculation unit that determines, as a reflection coefficient, a ratio between complex amplitudes of an incident wave and a reflected wave, the incident wave being transmitted to the first probe through the first cable, the reflected wave being obtained from reflection of the incident wave on the first probe, and

determines, as a transmission coefficient, a ratio between complex amplitudes of the incident wave and a transmitted wave obtained through the medium between the first probe and the second probe; a calibration unit that calibrates the reflection coefficient and the transmission coefficient according to calibration factors determined by using the standard; and a processing unit that measures an amount of moisture contained in the medium on the basis of the calibrated reflection coefficient and the calibrated transmission coefficient.

The first probe, the second probe, and the standard may be configured as a measurement device in the same housing.

The coefficient calculation unit, the calibration unit, and the processing unit may be configured as a signal processing device in the same housing.

The measurement device and the signal processing device may be integrated in the same housing.

The measurement device and the signal processing device may be configured in separated different housings.

The plurality of measurement devices and the signal processing device may be connected to each other.

The signal processing device may be capable of radio communications.

In order to solve the problem, the present disclosure provides a measuring method for a measurement device including a first probe in which a first cable electrically connectable to a first connection cable is embedded;

a second probe in which a second cable electrically connectable to a second connection cable is embedded; and a standard that is fixed at a predetermined positional relationship with the first probe and the second probe even during the measurement, is electrically connectable to the first connection cable and the second connection cable when the measurement is not conducted, and is used for calibration of the measurement, the method including: a calculating step of transmitting incident waves to the standard through the first connection cable in a predetermined order and calculating calibration factors on the basis of measurement data sequentially measured through the second connection cable;
a coefficient calculating step of determining, as a reflection coefficient, a ratio between complex amplitudes of an incident wave and a reflected wave, the incident wave being transmitted to the first probe through the first cable, the reflected wave being obtained from reflection of the incident wave on the first probe, and
determining, as a transmission coefficient, a ratio between complex amplitudes of the incident wave and a transmitted wave obtained through the medium between the first probe and the second probe;
a calibrating step of calibrating the reflection coefficient and the transmission coefficient according to the calibration factors; and
a processing step of performing processing for measuring an amount of moisture contained in the medium on the basis of the calibrated reflection coefficient and the calibrated transmission coefficient.

[Brief Description of Drawings]

[Fig. 1]

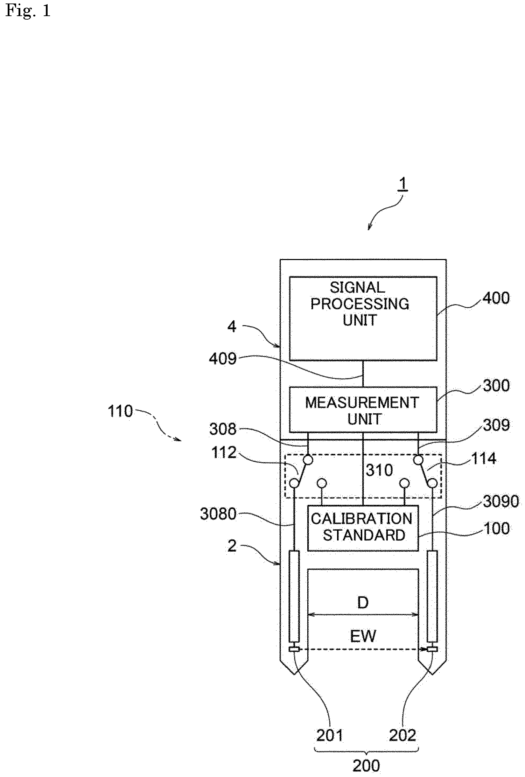

Fig. 1 is a block diagram illustrating a configuration example of a measurement device in a first embodiment.

[Fig. 2]

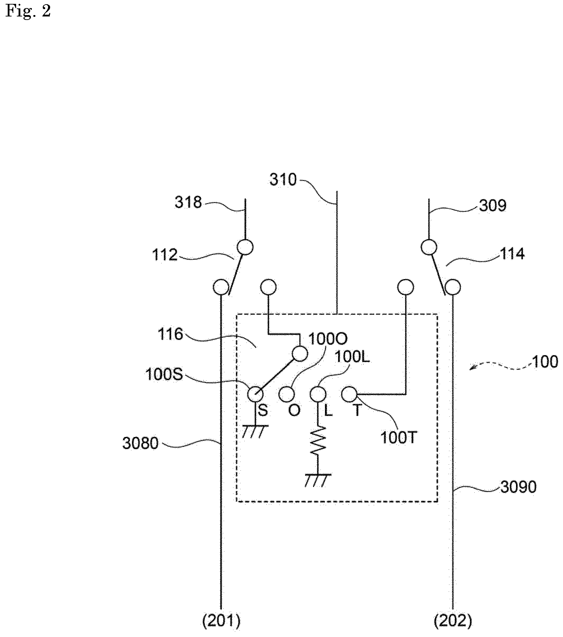

Fig. 2 illustrates a configuration example of a calibration standard according to the first embodiment.
[Fig. 3]

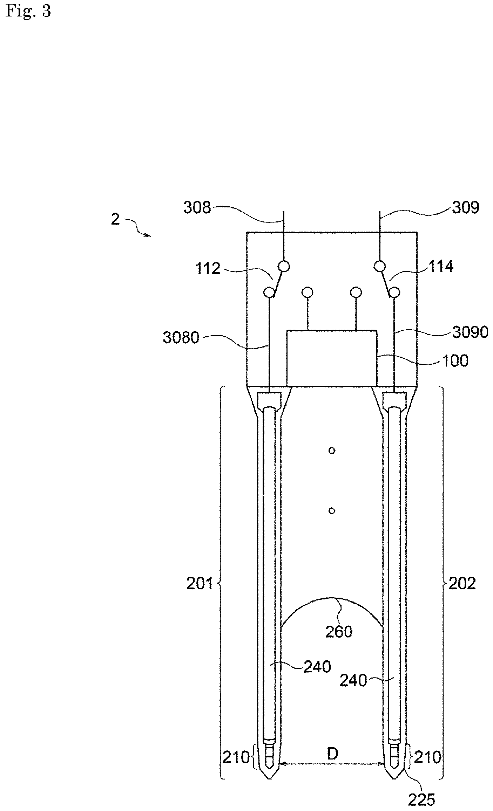

Fig. 3 is an external view of a sensor head according to the first embodiment.
[Fig. 4]

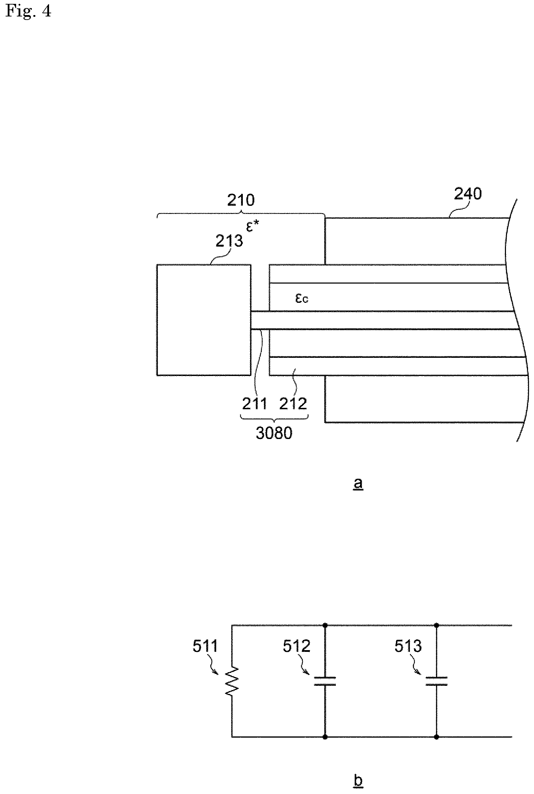

Fig. 4 illustrates an example of an antenna part and an equivalent circuit according to the first embodiment.
[Fig. 5]

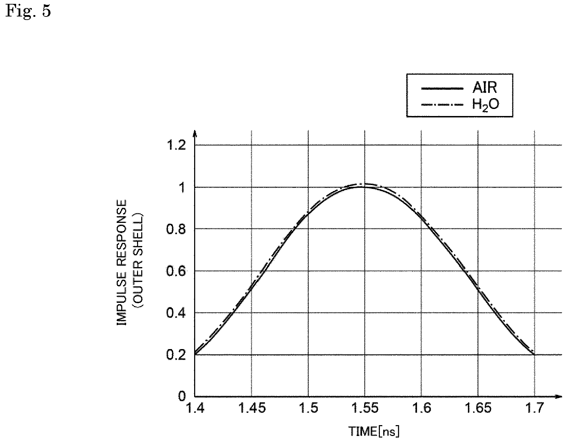

Fig. 5 is a graph showing an example of impulse response waveforms of a reflection coefficient according to the present embodiment.
[Fig. 6]

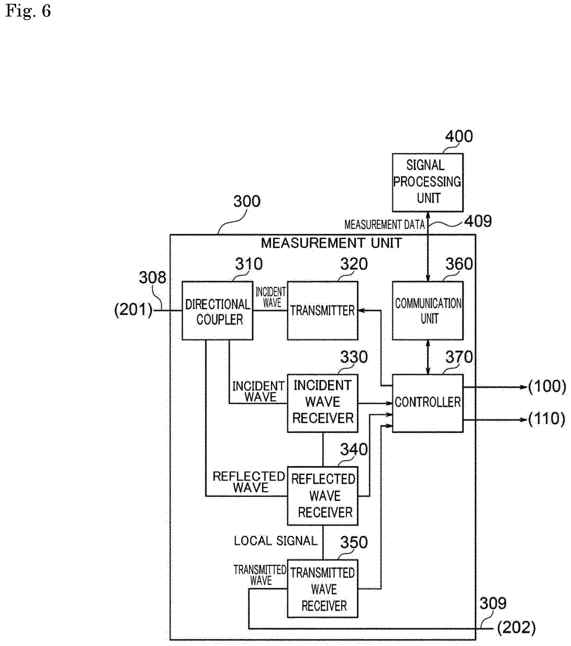

Fig. 6 is a block diagram illustrating a configuration example of a measurement unit according to the first embodiment.
[Fig. 7]

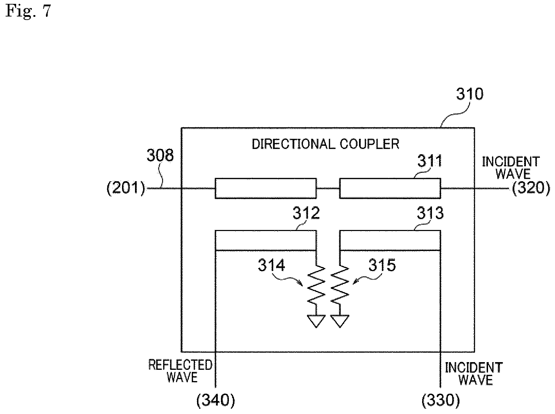

Fig. 7 illustrates a configuration example of a directional coupler according to the first embodiment.
[Fig. 8]

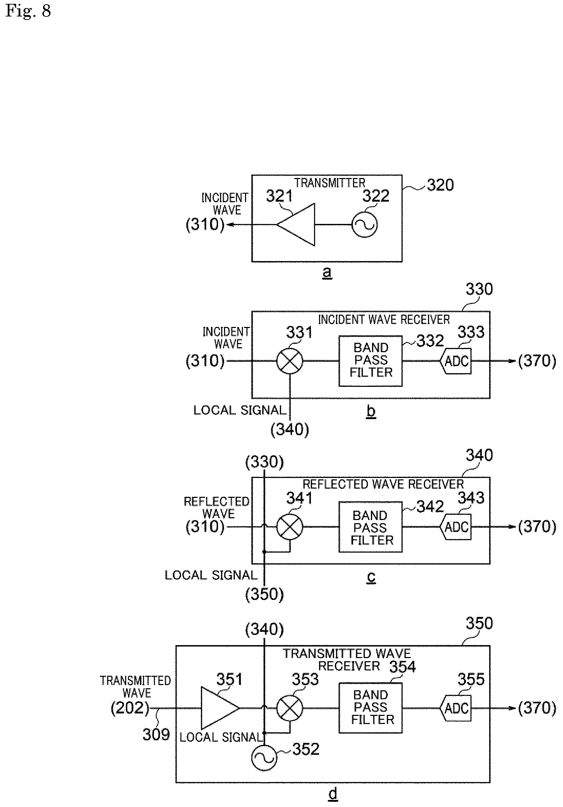

Fig. 8 is a circuit diagram illustrating a configuration example of a transmitter and a receiver according to the present embodiment.
[Fig. 9]

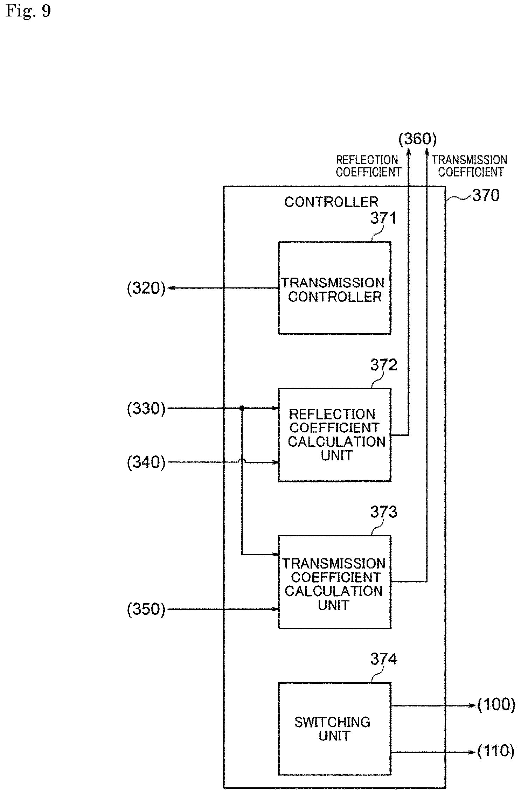

Fig. 9 is a block diagram illustrating a configuration example of a controller according to the first embodiment.
[Fig. 10]

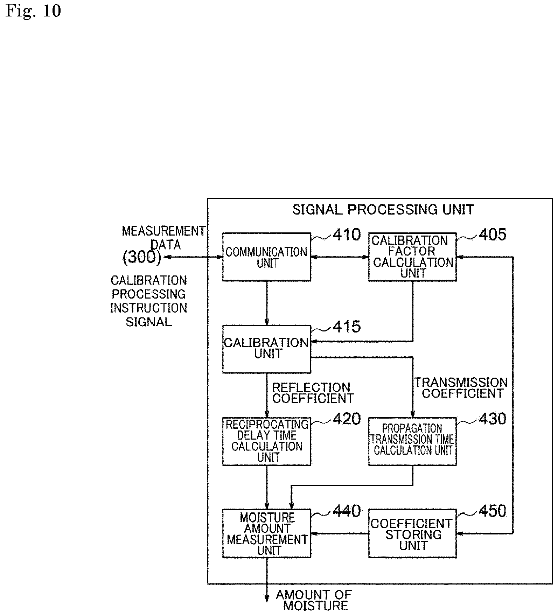

Fig. 10 is a block diagram illustrating a configuration example of a signal processing unit according to the first embodiment.
[Fig. 11]

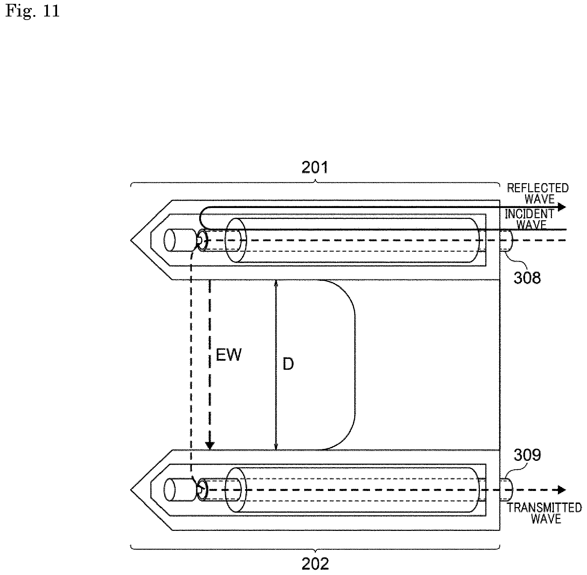

Fig. 11 is an explanatory drawing of a propagation path and a transmission path of electromagnetic waves and an electrical signal.
[Fig. 12]

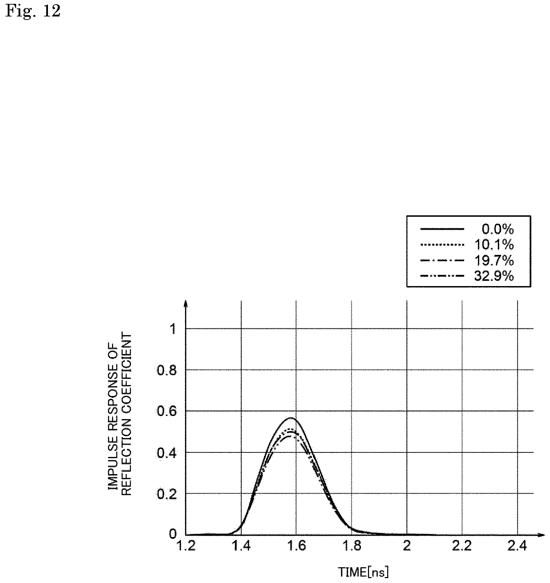

Fig. 12 is a graph showing an example of impulse response waveforms of a reflection coefficient according to the first embodiment.
[Fig. 13]

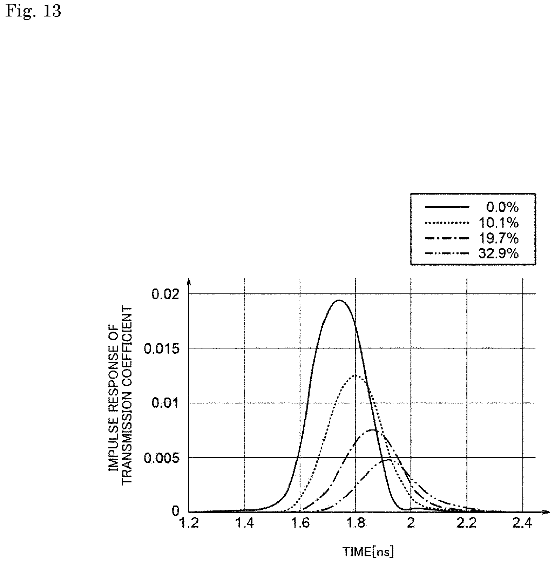

Fig. 13 is a graph showing an example of impulse response waveforms of a transmission coefficient according to the first embodiment.
[Fig. 14]

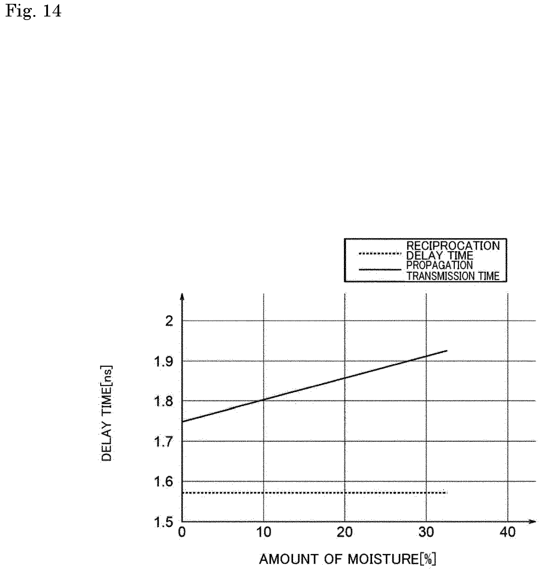

Fig. 14 is a graph showing an example of the relationship between a reciprocation delay time and a propagation transmission time and an amount of moisture.
[Fig. 15]

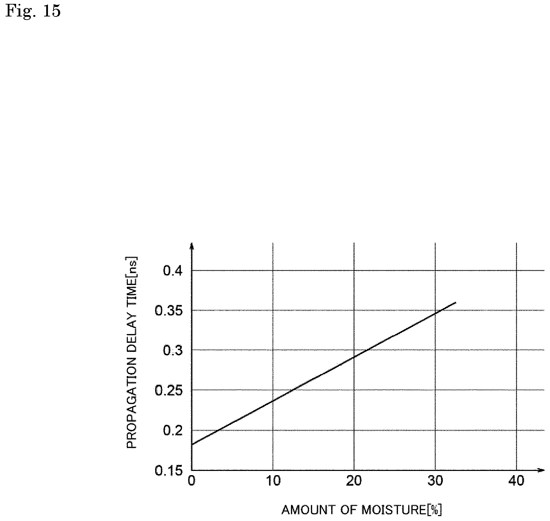

Fig. 15 is a graph showing an example of the relationship between a propagation delay time and an amount of moisture.
[Fig. 16]

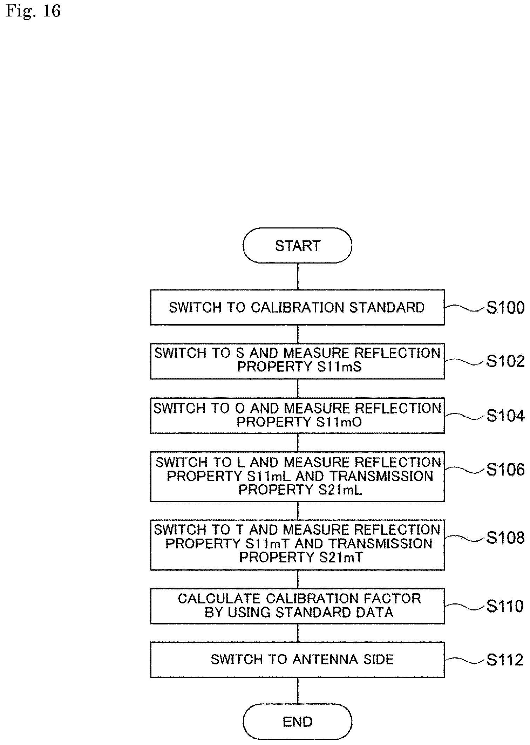

Fig. 16 is a flowchart showing a measuring operation example of a calibration factor.
[Fig. 17]

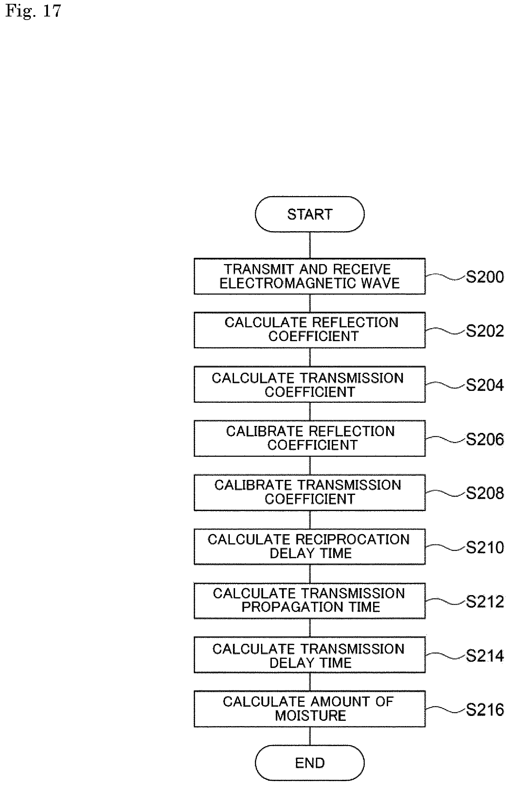

Fig. 17 is a flowchart showing an operation example of the measurement device.
[Fig. 18]

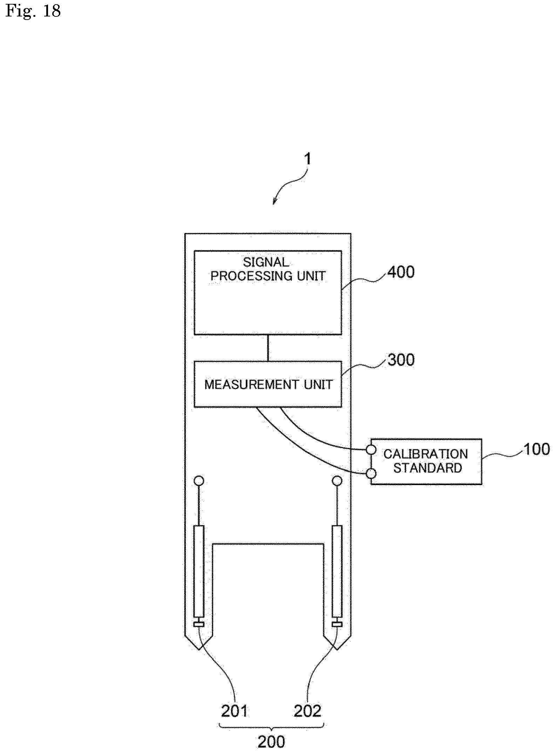

Fig. 18 illustrates a configuration example of a measurement device as a comparative example.
[Fig. 19]

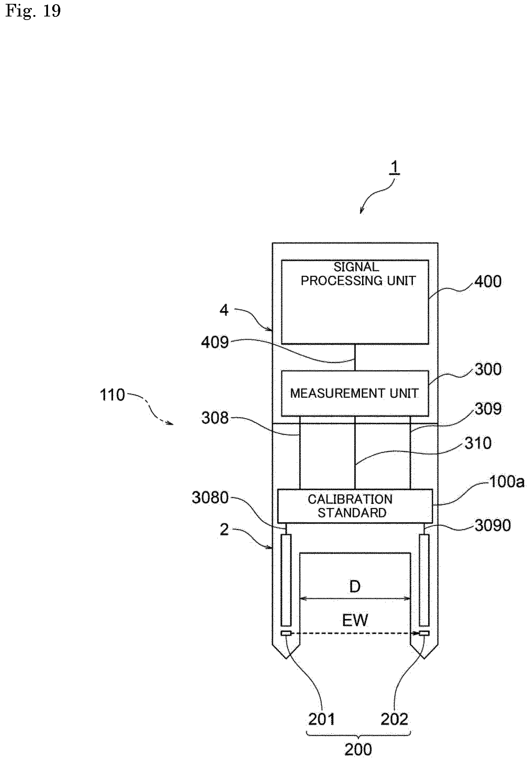

Fig. 19 illustrates a configuration example of a measurement device according to a second embodiment.
[Fig. 20]

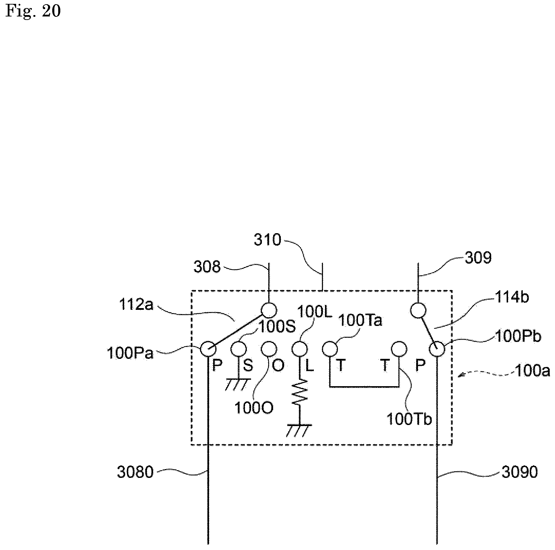

Fig. 20 illustrates a configuration example of a calibration standard according to the second embodiment.
[Fig. 21]

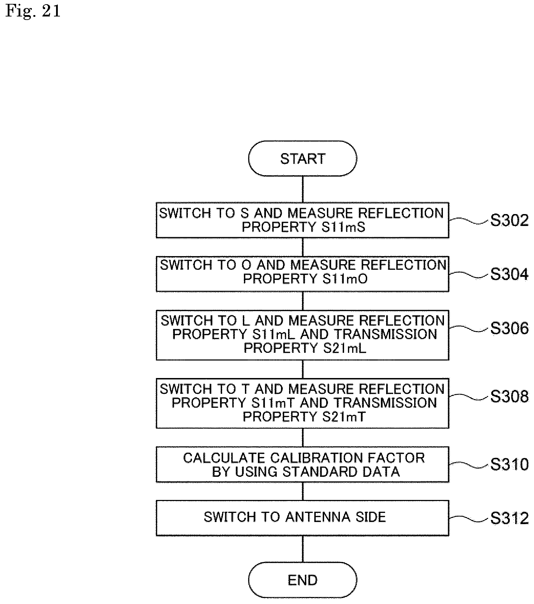

Fig. 21 is a flowchart showing a measuring operation example of a calibration factor according to the second embodiment.
[Fig. 22]

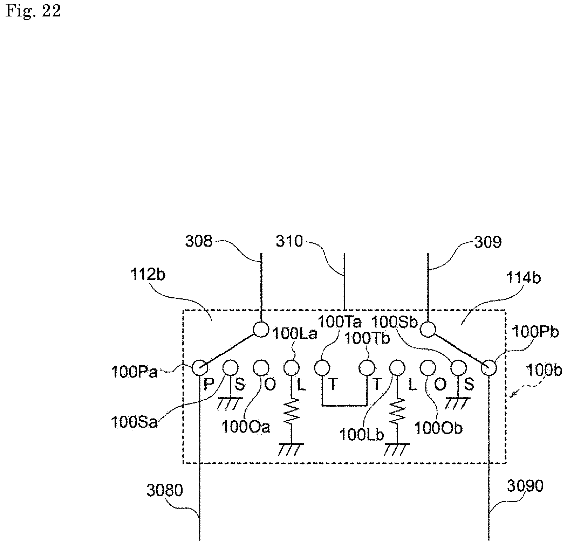

Fig. 22 illustrates a configuration example of a calibration standard according to a third embodiment.
[Fig. 23]

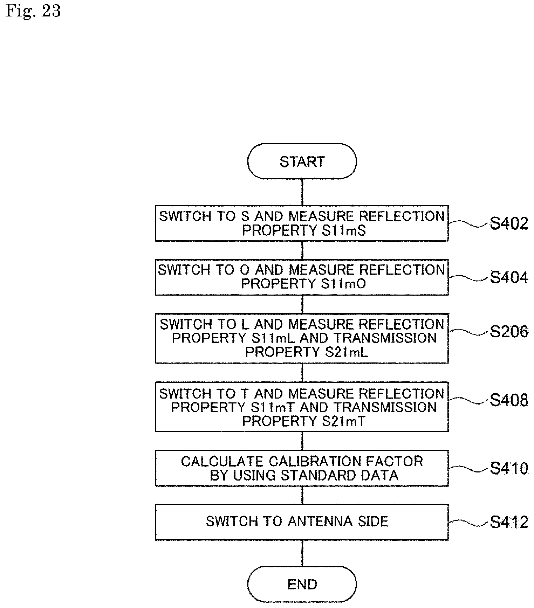

Fig. 23 is a flowchart showing a measuring operation example of a calibration factor according to the third embodiment.
[Fig. 24]

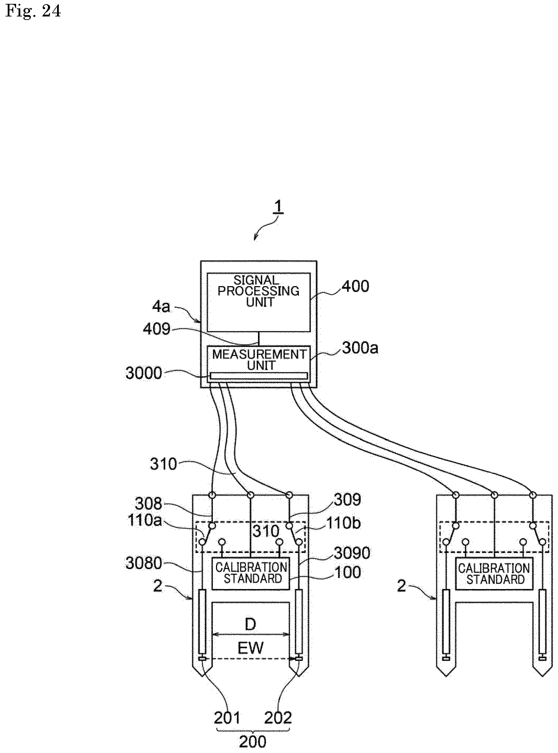

Fig. 24 illustrates a configuration example of a measurement device according to a fourth embodiment.
[Fig. 25]

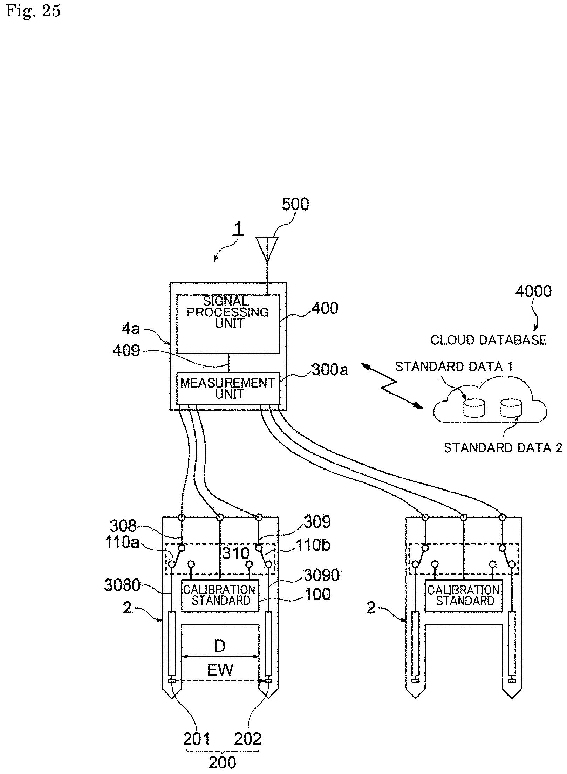

Fig. 25 illustrates a configuration example of a measurement device according to a fifth embodiment.

[Description of Embodiments]

Embodiments of a measurement device and a measurement method will be described below with reference to the drawings. Hereinafter, the main components of the measurement device will be mainly described. The measurement device may have components or functions that are not illustrated or described. The following description does not exclude components or functions that are not illustrated or described.

<First Embodiment>

[Configuration example of measurement device]

Fig. 1 is a block diagram illustrating a configuration example of a measurement device 1 according to a first embodiment of the present technique. The measurement device 1 measures an amount of moisture contained in a medium M and includes a sensor device 2 and a signal processing device 4 that are integrated in the same housing. The medium M is assumed to be, for example, soil for growing crops.

The sensor device 2 is configured to acquire, as measurement data, data necessary for measuring an amount of moisture. The sensor device 2 includes a calibration standard 100, a switching unit 110, and a sensor head 200. In addition, the signal processing device 4 includes a measurement unit 300 and a signal processing unit 400.

For example, the calibration standard 100 has four standards: short, open, load, and through. The calibration standard 100 is usable for, for example, so-called SOLT (Short-Open-Load-Thru) calibration. In the present embodiment, SOLT calibration is used for calibration. Calibration to be used is not limited thereto.

The switching unit 110 is, for example, a switching element configured with a semiconductor chip. The switching unit 110 includes a first switching unit 112 and a second switching unit 114. The first switching unit 112 is controlled by the control signal of the signal processing device 4 and switches connection between a first connection cable 308 and a first probe 201 or the calibration standard 100. The second switching unit 114 is controlled by the control signal of the signal processing device 4 and switches connection between a second connection cable 309 and a second probe 202 or the calibration standard 100.

As illustrated in Fig. 1, the sensor head 200 is a part composed of probes 201 and 202. The probe 201 is connected to the measurement unit 300 via a first cable 3080, the first switching unit 112, and the first connection cable 308. The probe 202 is connected to the measurement unit 300 via a second cable 3090, the first switching unit 112, and the second connection cable 309. For example, coaxial cables are used as the first cable 3080, the first connection cable 308, and the second cable 3090. The first cable 3080 and the second cable 3090 are connected to the probes 201 and 202 with the tips embedded in the probes 201 and 202, respectively.

The measurement unit 300 causes one of the probes 201 and 202 to transmit electromagnetic waves EW and causes the other to receive the electromagnetic waves EW to generate measurement data. The measurement unit 300 communicate with the calibration standard 100 via a signal line 310. The measurement unit 300 transmits measurement data to the signal processing unit 400 via a signal line 409. The signal processing unit 400 measures the amount of moisture using the measurement data.

The measurement unit 300 and the signal processing unit 400 are mounted on different semiconductor chips. The circuits of the measurement unit 300 and the signal processing unit 400 can be mounted on the same semiconductor chip as will be described later.

Furthermore, the measurement unit 300 may be configured with an electronic circuit board including a wiring layer and a semiconductor chip mounted on the electronic circuit board. The measurement unit 300 may include the electronic circuit board, the semiconductor chip, and a housing accommodating the electronic circuit board and the semiconductor chip. The cables 308 and 309 may be connected to the semiconductor chip via the wiring layer included in the electronic circuit board.

The measurement unit 300 including the electronic circuit board and the semiconductor chip or a housing accommodating the measurement unit 300 may have (1) a size within a substantially rectangular shape with one side measuring, for example, 1 to 20 centimeters (cm) in the extending direction (the board planar direction of the electronic circuit board) and the other side measuring 1 to 40 centimeters (cm) in a direction perpendicular to the one side, and have (2) a thickness of, for example, 2 to 20 millimeters (mm).

The measurement unit 300 can be disposed in at least one of two directions. Specifically, (1) the measurement unit 300 may be disposed such that the extending direction of the measurement unit 300 is parallel to the extending direction of the probes 201 and 202 or (2) the measurement unit 300 may be disposed such that the extending direction of the measurement unit 300 is perpendicular to the extending direction of the probes 201 and 202.

If the sensor device 2 and the signal processing device 4 are disposed in different housings, a system including the sensor device 2 and the signal processing device 4 can be handled as a measurement system.

[Configuration example of calibration standard]

Fig. 2 illustrates a configuration example of the calibration standard 100 according to the first embodiment of the present technique. The calibration standard 100 includes, for example, the four standards of short, open, load, and through and a third switching unit 116. Furthermore, the calibration standard 100 includes a first terminal 100S for connection to a short-circuit standard, a second terminal 100O for connection to an open standard, a third terminal 100L for connection to a reflection-free termination (load standard), and a fourth terminal 100T for direct connection to the first connection cable 308 and the second connection cable 309.

The third switching unit 116 is, for example, a switching element configured with a semiconductor chip. The third switching unit 116 is controlled by the control signal of the signal processing device 4 and switches connection between the first terminal 100S, the second terminal 100O, the third terminal 100L, and the fourth terminal 100T and the cable 308. The method of calibration will be specifically described later.

[Configuration example of sensor head]

Fig. 3 illustrates an external view of the sensor head 200 according to the first embodiment of the present technique. The sensor head 200 includes the probes 201 and 202. The calibration standard 100, the first switching unit 112, and the second switching unit 114 are disposed above the sensor head 200.

The probes 201 and 202 are, for example, 75 to 150 millimeters (mm) in length. The probes 201 and 202 are, for example, 3 to 30 millimeters (mm) in thickness (diameter or width in the cross sections of the probes). These probes 201 and 202 are disposed in a medium such as soil and each include an antenna part 210 capable of transmitting and receiving electromagnetic waves at a predetermined frequency between the probes 201 and 202.

The probes 201 and 202 are embedded in the medium such that a distance between the antenna parts 210 has a predetermined value D. For example, these probes are embedded in almost vertical positions in the medium. When the distance between the antenna parts 210 is D, the positions of the probes are not limited to vertical positions.

The antenna parts 210 are provided at or near the tips (in other words, ends) of the probes 201 and 202 and are configured to transmit and receive electromagnetic waves. Although the antenna parts 210 are provided at the tips of the probes 201 and 202, it is not limited to this configuration. For example, the antenna parts 210 can also be provided at the central positions of the probes 201 and 202, respectively.

Furthermore, the antenna parts 210 are configured with micro antennas that are sized not to resonate the probes 201 and 202. This can suppress a decrease in measurement accuracy due to the resonance of the probes 201 and 202.

As described above, the tips of the cables 308 and 309 (coaxial cables) in Fig. 1 are embedded in the probes 201 and 202, respectively. Parts of the coaxial cables are opened and are used as the antenna parts 210. The outer peripheries of parts of the coaxial cables other then the antenna parts 210 are covered with an electromagnetic wave absorbing material 240. The electromagnetic wave absorbing material 240 can suppress the leakage of electromagnetic waves from regions other than the opening parts.

ANi-Zn based ferrite is mainly used as the electromagnetic wave absorbing material 240 but the present technique is not limited thereto. Other materials having high dielectric constants, e.g., sendust and permalloy may be used depending on the frequency of the electromagnetic waves EW. The electromagnetic wave absorbing material 240 may be omitted as necessary or may be provided for only one of the probes 201 and 202.

The distance D between the antenna parts 210 is not particularly limited. When the distance D is too long, the attenuation of the electromagnetic waves EW propagating through the medium M may increase and prevent the acquisition of sufficient intensity of reception. When the distance D is too short, observation may become technically difficult. In consideration of these circumstances, the distance D is set at a proper value. For example, the distance D is 25 to 75 millimeters (mm).

Between the probes 201 and 202, a spacer 260 is disposed to determine a distance between the antenna parts 210. The outer peripheries of the probes 201 and 202 are each covered with an outer shell 225 having a thickness of 1 to 3 millimeters (mm) and are separated from the medium. The spacer 260 and the outer shell 225 are made of an electromagnetic wave transmissive material. Examples of the electromagnetic wave transmissive material include inorganic materials such as polymeric materials, glass, and PTEF (PolyTEtraFluoroethylene). As the polymeric materials, PC (PolyCarbonate), PES (PolyEtherSulfone), PEEK (PolyEtherEtherKetone), PSS (PolyStyrene Sulfonic acid), and the like are used. Other polymeric materials such as PMMA (PolyMethylMethAcrylate) and PET (PolyEthylene Terephthalate) are also used.

The thickness of the spacer 260 may be smaller than the size and thickness of the measurement unit 300 including the electronic circuit board and the semiconductor chip. For example, when the measurement unit 300 is disposed such that the extending direction of the measurement unit 300 is parallel to the extending direction of the probes 201 and 202, the thickness of the spacer 260 may be smaller than the thickness of the measurement unit 300, preferably smaller than a half of the thickness of the measurement unit 300, and more preferably smaller than one third of the thickness of the measurement unit 300. Alternatively, when the measurement unit 300 is disposed such that the extending direction of the measurement unit 300 is perpendicular to the extending direction of the probes 201 and 202, the thickness of the spacer 260 may be smaller than the length of the measurement unit 300 in one extending direction of the measurement unit 300, preferably smaller than a half of the length of the measurement unit 300, and more preferably smaller than one third of the length of the measurement unit 300. Moreover, the thickness of the spacer 260 may be smaller than the thickness of at least one of the probes 201 and 202 (diameter or width in the cross sections of the probes), preferably smaller than a half of the thickness of the probe, more preferably smaller than one third of the thickness of the probe. For example, the spacer 260 may have a thickness of 1 to 3 millimeters (mm).

The configuration in which the thickness of the spacer 260 is smaller than the thickness of the measurement unit 300, smaller than the length of the measurement unit 300 in one extending direction thereof, or smaller than the thickness of at least any one of the probes 201 and 202 (diameter or the width of the probe cross section) is effective in a moisture sensor that measures a propagation delay time of electromagnetic waves between antennas. Even if the spacer 260 is made of an electromagnetic wave transmissive material, electromagnetic waves radiated from the transmitting antenna may be reflected by the spacer and received by the receiving antenna, resulting in noise depending on the material. By setting the thickness of the spacer 260 according to the foregoing configuration, the noise reflected by the spacer 260 can be reduced as compared to configurations other than the foregoing configuration. The effect of suppressing noise by reducing the thickness of the spacer is not produced in moisture sensors other than a moisture sensor that measures a propagation delay time of electromagnetic waves between antennas. The effect is produced because a moisture sensor measures a propagation delay time of electromagnetic waves between antennas.

A distance d from the antenna parts (210 and 220) of the pair of probes 201 and 202 to the lower end of the spacer 260 is preferably larger than the distance D between the antennas. The distance d, in particular, is preferably larger than twice the distance D between the antennas. Furthermore, the distance d is more preferably larger than three times the distance D between the antennas and smaller than the lengths of the probes 201 and 202. Even if the spacer 260 between the probes 201 and 202 is made of an electromagnetic wave transmissive material, micro waves radiated from the transmitting antenna may be reflected by the spacer and received by the receiving antenna, resulting in noise depending on the material. As described above, the noise can be reduced by separating the spacer 260 from the antennas. This "effect of reducing the noise by separating the spacer 260 from the antennas" is not produced in moisture sensors other than a moisture sensor using a method of measuring a propagation delay time between antennas. The effect is produced because a moisture sensor uses a method of measuring a propagation delay time between antennas as in the present technique.

Moreover, the outer edge of the spacer 260 extends between the pair of probes (201 and 202) and has an arc shape near the antenna parts (210 and 220) as illustrated in Fig. 3. The outer edge shaped like an arc instead of a straight line can further reduce noise caused by micro waves that are radiated from the antenna, reflected by the spacer 260, and received by the receiving antenna. This "effect of reducing noise by the arc-shaped spacer 260" is not produced in moisture sensors other than a moisture sensor using a method of measuring a propagation delay time between antennas. The effect is produced because a moisture sensor uses a method of measuring a propagation delay time between antennas.

[Configuration example of antenna part]

Fig. 4 illustrates an example of the antenna part 210 and an equivalent circuit according to the first embodiment of the present technique. In Fig. 4, a is an enlarged view of the antenna part 210. In Fig. 4, b is an example of the equivalent circuit of the antenna part 210.

The cable 3080 (coaxial cable or the like) embedded in the probe 201 includes a core wire part 211 and a shield part 212. The thickness and the length of the cable are not particularly limited and can be set to any thickness and length. As illustrated in a of Fig. 4, the core wire part 211 is configured with a copper wire and the shield part 212 is configured with a copper pipe. The shield part 212 may be configured with a copper wire mesh.

Apart around the tip of the cable 3080 (coaxial cable or the like) is opened and an electrode part 213 is attached to the opening. Accordingly, the antenna parts 210 of the probes 201 and 202 serve as micro-dipole antennas having a length of about 4 to 10 millimeters (mm). The opening has an opening shape, e.g., a square, a circle, an oval, or an ellipse. The major axis of the opening can be appropriately set according to the wavelength of electromagnetic waves to be used.

In addition, as illustrated in b of Fig. 4, the equivalent circuit of the antenna part 210 is represented as a circuit in which a resistor 511 and fringing capacitors 512 and 513 are connected in parallel. The capacitance value of the fringing capacitor 512 is a value corresponding to a dielectric constant $\varepsilon c$ of a material spreading in the coaxial cable. The capacitance value of the fringing capacitor 513 is a value corresponding to a dielectric constant $\varepsilon^*$ of a material spreading around the electrode 513.

When an electrical signal is transmitted to one of the probes 201 and 202, a part of the signal is reflected at the end of the probe and thus the electrical signal reciprocates in the coaxial cable. As for this electrical signal, a wave in the input signal is assumed to be an "incident wave" and a wave obtained from reflection of the incident wave is assumed to be a "reflected wave".

A comparative example is conceived in the absence of the outer shell 225. In this comparative example, a reciprocation delay time for the reciprocation of an electrical signal in the coaxial cable is changed by a temperature and the dielectric constant $\varepsilon^*$ of the medium.

Since the coaxial cable is extended by thermal expansion as the temperature increases, the delay time increases. When the dielectric constant $\varepsilon^*$ of the medium changes, the fringing capacitor 512 changes according to the value and the peak time of an impulse response of a reflection coefficient changes. In this case, the reflection coefficient is a ratio between the complex amplitudes of an incident wave and a reflected wave.

Fig. 5 is a graph showing an example of the impulse response waveforms of a reflection coefficient according to the present embodiment. In Fig. 5, the vertical axis represents an impulse response of the reflection coefficient and the horizontal axis represents a time. A solid-line curve represents an impulse response waveform when a medium is the air, and a chain-line curve represents an impulse response waveform when a medium is water. As shown in Fig. 5, the peak value of the impulse response does not fluctuate. Accordingly, the reciprocation delay time can be calculated with high accuracy.

Fig. 6 is a block diagram illustrating a configuration example of the measurement unit 300 according to the first embodiment of the present technique. The measurement unit 300 includes a directional coupler 310, a transmitter 320, an incident wave receiver 330, a reflected wave receiver 340, a transmitted wave receiver 350, a communication unit 360, and a controller 370. For example, a vector network analyzer is used as the measurement unit 300.

The directional coupler 310 separates an electrical signal transmitted through the cable 308 into an incident wave and a reflected wave. The incident wave is a wave of an electrical signal transmitted from the transmitter 320 and the reflected wave is obtained from the reflection of the incident wave at the end of the probe 201. The directional coupler 310 provides the incident wave to the incident wave receiver 330 and provides the reflected wave to the reflected wave receiver 340.

The transmitter 320 is adapted to transmit the electrical signal at a predetermined frequency as a transmission signal to the probe 201 via the directional coupler 310 and the cable 308. As the incident waves in the transmission signal, for example, continuous waves (CW) are used. The transmitter 320 sequentially switches frequencies in steps of 50 megahertz (MHz) within the frequency band of 1 to 9 gigahertz (GHz) and transmits the transmission signal.

The incident wave receiver 330 receives the incident wave from the directional coupler 310. The reflected wave receiver 340 receives the reflected wave from the directional coupler 310. The transmitted wave receiver 350 receives a transmitted wave from the probe 202. In this case, the transmitted wave is obtained by converting an electromagnetic wave, which is transmitted through the medium between the probes 201 and 202, into an electrical signal by the probe 202.

The incident wave receiver 330, the reflected wave receiver 340, and the transmitted wave receiver 350 perform quadrature detection and AD (Analog to Digital) conversion on the received incident wave, reflected wave, and transmitted wave and provide the resultant waves to the controller 370 as reception data.

The incident wave receiver 330, the reflected wave receiver 340, and the transmitted wave receiver 350 are examples of a receiver in the claims.

The controller 370 controls the transmitter 320 to transmit the transmission signal including the incident wave and performs processing for determining a reflection coefficient and a transmission efficient. As described above, the controller 370 controls the first switching unit 112, the second switching unit 114, and the third switching unit 116. As described above, the reflection coefficient is a ratio between the complex amplitudes of the incident wave and the reflected wave. The transmission coefficient is a ratio between the complex amplitudes of the incident wave and the transmitted wave. The controller 370 provides the determined reflected coefficient and transmission coefficient to the communication unit 360.

The communication unit 360 transmits data representing the reflection coefficient and the transmission coefficient as measurement data to the signal processing unit 400 through a signal line 409.

[Configuration example of directional coupler]

Fig. 7 illustrates a configuration example of the directional coupler 310 according to the first embodiment of the present technique. The directional coupler 310 includes transmission lines 311, 312, and 313 and terminating resistors 314 and 315. The directional coupler 310 can be implemented as, for example, a bridge coupler suitable for miniaturization.

One end of the transmission line 311 is connected to the transmitter 320 and the other end is connected to the probe 201 via the cable 308. The transmission line 312 is shorter than the transmission line 311 and is a line coupled to the transmission line 311 through electromagnetic field coupling. One end of the transmission line 312 is connected to the terminating resistor 314 and the other end is connected to the reflected wave receiver 340. The transmission line 313 is shorter than the transmission line 311 and is a line coupled to the transmission line 311 through electromagnetic field coupling. One end of the transmission line 313 is connected to the terminating resistor 315 and the other end is connected to the incident wave receiver 330.

In the foregoing configuration, the directional coupler 310 separates an electrical signal into an incident wave and a reflected wave and provides the incident wave and the reflected wave to the incident wave receiver 330 and the reflected wave receiver 340.

[Configuration example of transmitter and receiver]

Fig. 8 is a circuit diagram illustrating a configuration example of the transmitter 320 and the receivers according to the present embodiment. In Fig. 8, a is a circuit diagram illustrating a configuration example of the transmitter 320 and b is a circuit diagram illustrating a configuration example of the incident wave receiver 330. In Fig. 8, c is a circuit diagram illustrating a configuration example of the reflected wave receiver 340 and d is a circuit diagram illustrating a configuration example of the transmitted wave receiver 350.

As illustrated in a of Fig. 8, the transmitter 320 includes a transmission signal oscillator 322 and a driver 321.

The transmission signal oscillator 322 is adapted to generate an electrical signal as a transmission signal under the control of the controller 370. The driver 321 is adapted to output the transmission signal to the directional coupler 310. For example, the transmission signal S(t) is represented by the following formula:

$$S(t) = |A| \cos(2\pi ft + \theta)$$

In the above formula, t represents a time and the unit is, for example, nanoseconds (ns). |A| indicates the amplitude of the transmission signal. cos() indicates a cosine function. f indicates a frequency, and the unit is, for example, hertz (Hz). $\theta$ represents a phase, and the unit is, for example, radian (rad).

As illustrated in b of Fig. 8, the incident wave receiver 330 includes a mixer 331, a band pass filter 332, an analog-to-digital converter 333.

The mixer 331 performs quadrature detection by mixing two local signals having a phase difference of 90 degrees and the transmission signal. A complex amplitude composed of an in-phase component II and a quadrature component QI is obtained by the quadrature detection. These in-phase component II and quadrature component QI are represented by the following formulas: The mixer 331 provides the complex amplitude to the analog-to-digital converter 333 through the band pass filter 332.

$$II = |A| \cos(\theta)$$

$$QI = |A| \sin(\theta)$$

In the above formulas, sin() represents a sine function.

The band pass filter 332 passes a component of a predetermined frequency band. The analog-to-digital converter 333 performs AD conversion. The analog-to-digital converter 333 generates data representing the complex amplitude according to AD conversion and provides the data to the controller 370 as reception data.

As illustrated in c of Fig. 8, the reflected wave receiver 340 includes a mixer 341, a band pass filter 342, and an analog-to-digital converter 343. The configurations of the mixer 341, the band pass filter 342, and the analog-to-digital converter 343 are identical to those of the mixer 331, the band pass filter 332, and the analog-to-digital converter 333. The reflected wave receiver 340 performs quadrature detection on a reflected wave to acquire a complex amplitude composed of an in-phase component IR and a quadrature component QR and provides reception data representing the complex amplitude to the controller 370.

As illustrated in d of Fig. 8, the transmitted wave receiver 350 includes a receiver 351, a local signal oscillator 352, a mixer 353, a band pass filter 354, and an analog-to-digital converter 355. The configurations of the mixer 353, the band pass filter 354, and the analog-to-digital converter 355 are identical to those of the mixer 331, the band pass filter 332, and the analog-to-digital converter 333.

The receiver 351 receives an electrical signal including a transmitted wave through the cable 309 and outputs the transmitted wave to the mixer 353. The local signal oscillator 352 generates two local signals having a phase difference of 90 degrees.

The transmitted wave receiver 350 performs quadrature detection on the transmitted wave to acquire a complex amplitude composed of an in-phase component IT and a quadrature component QT and provides data representing the complex amplitude to the controller 370 as reception data.

The circuits of the transmitter 320 and the receivers (incident wave receiver 330 or the like) are not limited to the circuits illustrated in Fig. 8 if the circuits can transmit and receive an incident wave or the like.

[Configuration example of controller]

Fig. 9 is a block diagram illustrating a configuration example of the controller 370 according to the first embodiment of the present technique. The controller 370 includes a transmission controller 371, a reflection coefficient calculation unit 372, a transmission coefficient calculation unit 373, and a switching unit 374.

The transmission controller 371 controls the transmitter 320 to transmit a transmission signal.

The reflection coefficient calculation unit 372 calculates a reflection coefficient $\Gamma$ for each frequency. The reflection coefficient calculation unit 372 receives the complex amplitudes of an incident wave and a reflected wave from the incident wave receiver 330 and the reflected wave receiver 340 and calculates a ratio between the complex amplitudes as a reflection coefficient $\Gamma'$ according to the following formula:

$$\Gamma' = (IR + jQR)/(II + jQI) \ \ldots \ (1)$$

In the above formula, j is an imaginary unit.

The reflection coefficient calculation unit 372 calculates reflection coefficients with respect to N (N is an integer) frequencies f1 to fN according to Formula 1. These N reflection coefficients are denoted as $\Gamma 1$ to $\Gamma N$. The reflection coefficient calculation unit 372 provides the reflection coefficients to the communication unit 360.

The transmission coefficient calculation unit 373 calculates a transmission coefficient T' for each frequency. The transmission coefficient calculation unit 373 receives complex amplitudes of an incident wave and a transmitted wave from the incident wave receiver 330 and the transmitted wave receiver 350 and calculates a ratio between the complex amplitudes as a transmission coefficient T' according to the following formula:

$$T' = (IT + jQT)/(II + jQI) \ \ldots \ (2)$$

The transmission coefficient calculation unit 373 calculates transmission coefficients with respect to the N frequencies f1 to fN according to Formula 2. These N reflection coefficients are denoted as T'1 to T'N. The transmission coefficient calculation unit 373 provides the transmission coefficients to the signal processing unit 400 through the communication unit 360.

When the amount of moisture contained in the medium is measured, the switching unit 374 performs control to switch electrical connection between the first switching unit 112 and the second switching unit 114 to electrical connection between the probe 201 and the probe 202. When calibration factors are measured, the switching unit 374 performs control to switch electrical connection to the first switching unit 112 and the second switching unit 114 to the calibration standard 100 in a predetermined order. Moreover, when the calibration factors are measured, the switching unit 374 performs control to switch the electrical connection of the third switching unit 116 to the first terminal 100S, the second terminal 100O, the third terminal 100L, and the fourth terminal 100T in a predetermined order.

[Configuration example of signal processing unit]

Fig. 10 is a block diagram illustrating a configuration example of the signal processing unit 400 according to the first embodiment of the present technique. The signal processing unit 400 includes a calibration factor calculation unit 405, a communication unit 410, a calibration unit 415, a reciprocation delay time calculation unit 420, a propagation transmission time calculation unit 430, a moisture amount measurement unit 440, and a coefficient storing unit 450.

The calibration factor calculation unit 405 calculates calibration factors (ED, ERX, ES, EX, ET) according to, for example, SOLT (Short-Open-Load-Thru) calibration. The method for calculating the calibration factors (ED, ERX, ES, EX, ET) will be specifically described later. The calibration factor calculation unit 405 provides the calculated calibration factors (ED, ERX, ES, EX, ET) to the coefficient storing unit 450.

The communication unit 410 receives measurement data from the measurement unit 300. The communication unit 410 provides reflection coefficients $\Gamma'1$ to $\Gamma'N$ in the measurement data and transmission coefficients T'1 to T'N to the calibration unit 415.

On the basis of the calibration factors (ED, ERX, ES, EX, ET) stored in the coefficient storing unit 450, the calibration unit 415 provides the calibrated reflection coefficients $\Gamma1$ to $\Gamma N$ to the reciprocation delay time calculation unit 420 and provides the calibrated transmission coefficients T1 to TN to the propagation transmission time calculation unit 430.

The reciprocation delay time calculation unit 420 calculates the reciprocation time of an electrical signal in the cable 308 as a reciprocation delay time on the basis of the reflection coefficients. The reciprocation delay time calculation unit 420 determines an impulse response $h\Gamma(t)$ by performing inverse Fourier transformation on the reflection coefficients $\Gamma1$ to $\Gamma N$. The reciprocation delay time calculation unit 420 then determines, as a reciprocation delay time $\tau11$, a time difference between the timing of the peak value of the impulse response $h\Gamma(t)$ and the transmission timing of CW waves and provides the reciprocation delay time $\tau11$ to the moisture amount measurement unit 440.

The propagation transmission time calculation unit 430 calculates, as a propagation transmission time, a time for the propagation and transmission of electromagnetic waves and an electrical signal through the medium and the cables 308 and 309 on the basis of the transmission coefficients. The propagation transmission time calculation unit 430 determines the impulse response $hT(t)$ by performing inverse Fourier transformation on the transmission coefficients $T1$ to $TN$. The propagation transmission time calculation unit 430 then determines, as a propagation transmission time $\tau21$, a time difference between the timing of the peak value of the impulse response $hT(t)$ and the transmission timing of the CW waves and provides the propagation transmission time $\tau21$ to the moisture amount measurement unit 440.

The moisture amount measurement unit 440 measures the amount of moisture on the basis of the reciprocation delay time $\tau11$ and the propagation transmission time $\tau21$. First, the moisture amount measurement unit 440 calculates a propagation delay time $\tau d$ from the reciprocation delay time $\tau11$ and the propagation transmission time $\tau21$. In this case, the propagation delay time is a time during which electromagnetic waves propagate through the medium between the probes 201 and 202. The propagation delay time $\tau d$ is calculated by the following formula:

$$\tau d = \tau21 - \tau11 \ ... \ (3)$$

In the above formula, the units of the reciprocation delay time $\tau11$, the propagation transmission time $\tau21$, and the propagation delay time $\tau d$ are, for example, nanoseconds (ns).

The moisture amount measurement unit 440 then reads coefficients a and b representing the relationship between the amount of moisture and the propagation delay time $\tau d$ from the coefficient storing unit 450 and measures the amount of moisture x by substituting the propagation delay time $\tau d$, which is calculated by Formula 3, into the formula below. Furthermore, the moisture amount measurement unit 440 outputs the measured amount of moisture to an external device or apparatus as necessary.

$$\tau d = a\ x + b \ ... \ (4)$$

In the above formula, the unit of the amount of moisture x is, for example, percent by volume (%).

The coefficient storing unit 450 stores the calibration factors (ED, ERX, ES, EX, ET) and the coefficients a and b. A nonvolatile memory is used as the coefficient storing unit 450.

Fig. 11 is an explanatory drawing of a propagation path and a transmission path of electromagnetic waves and an electrical signal according to the first embodiment of the present technique.

As described above, the transmitter 320 transmits an electrical signal including an incident wave to the probe 201 as a transmission signal through the cable 308 having the tip embedded in the probe 201.

The incident wave is reflected at the end of the probe 201 and the reflected wave is received by the reflected wave receiver 340. Thus, the electrical signal including the incident wave and the reflected wave reciprocates in the cable 308. In Fig. 11, an arrow of a thick solid line indicates a path for the reciprocation of the electrical signal in the cable 308. A time during which the electrical signal reciprocates through the path corresponds to the reciprocation delay time $\tau11$.

Also, the electrical signal including the incident wave is converted into the electromagnetic waves EW by the probe 201, and the electromagnetic waves EW pass through (in other words, propagate through) the medium between the probes 201 and 202. The probe 202 converts the electromagnetic waves EW into an electrical signal. The transmitted wave receiver 350 receives the transmitted waves in the electrical signal via the cable 309. In other words, the electrical signal including the incident waves is transmitted through the cable 308, is converted into the electromagnetic waves EW, is propagated through the medium, is converted into the electrical signal including the transmitted waves, and is transmitted through the cable 309. An arrow of a thick dotted line in Fig. 11 indicates a path for propagating and transmitting the electromagnetic waves and the electrical signal (the incident waves and the transmitted waves) through the medium and the cables 308 and 309. A time during which the electromagnetic waves and the electrical signal are propagated and transmitted through the path corresponds to the propagation transmission time $\tau21$.

The controller 370 in the measurement unit 300 determines the reflection coefficient $\Gamma$ and the transmission coefficient T according to Formula 1 and Formula 2. The signal processing unit 400 then determines the reciprocation delay time τ11 and the propagation transmission time τ21 from the reflection coefficient Γ and the transmission coefficient T.

In this case, a path from the transmission of the incident wave to the reception of the transmitted wave includes the medium and the cables 308 and 309. Thus, the propagation delay time τd during which the electromagnetic waves propagate through the medium is determined by a difference between the propagation transmission time τ21 and a delay time during which the electrical signal is transmitted through the cables 308 and 309. On the assumption that the cables 308 and 309 have the same length, a delay time of transmission through the cable 308 and a delay time of transmission through the cable 309 are equal to each other. In this case, the sum of the delay times during which the electrical signal is transmitted through the cables 308 and 309 is equal to the reciprocation delay time τ11 during which the electrical signal reciprocates through the cable 308. Thus, Formula 3 is established and the signal processing unit 400 can calculate the propagation delay time τd according to Formula 3.

The signal processing unit 400 then calculates a propagation transmission time from the determined reciprocation delay time τ11 and propagation transmission time τ21 and performs processing for measuring the amount of moisture contained in the medium from the propagation transmission time and the coefficients a and b.

Fig. 12 is a graph showing an example of impulse response waveforms of a reflection coefficient according to the first embodiment of the present technique. In Fig. 12, the vertical axis represents an impulse response of the reflection coefficient and the horizontal axis represents a time.

It is assumed that four types of Toyoura standard sand having different amounts of moisture are prepared as media and the measurement device 100 determines impulse responses of reflection coefficients. The amounts of moisture are 0.0, 10.1, 19.7, and 32.9 percents by volume (%).

As indicated in Fig. 12, the peak value of the reflection coefficient does not fluctuate even if the amount of moisture changes. In other words, the reciprocation delay time is kept constant. This is because the probes 201 and 202 are isolated by the outer shell 225 as described above.

Fig. 13 is a graph showing an example of impulse response waveforms of a transmission coefficient according to the first embodiment of the present technique. In Fig. 13, the vertical axis represents an impulse response of the transmission coefficient and the horizontal axis represents a time. In Fig. 13, media to be measured are four types of Toyoura standard sand as in Fig. 12.

As indicated in Fig. 13, the timing of the peak value of the transmission coefficient is delayed as the amount of moisture increases. Accordingly, the propagation transmission delay time increases with the amount of moisture.

Fig. 14 is a graph showing an example of the relationship between a reciprocation delay time and a propagation transmission time and an amount of moisture according to the first embodiment of the present technique. In Fig. 14, the vertical axis represents a reciprocation delay time or a propagation transmission time and the horizontal axis represents an amount of moisture.

In Fig. 14, a dotted line indicates the relationship between the reciprocation delay time obtained from Fig. 12 and the amount of moisture. In Fig. 14, a solid line indicates the relationship between the propagation transmission time obtained from Fig. 13 and the amount of moisture. As indicated in Fig. 14, the reciprocation delay time is constant irrespective of the amount of moisture. The propagation transmission delay time increases with the amount of moisture.

Fig. 15 is a graph showing an example of the relationship between a propagation delay time and an amount of moisture according to the first embodiment of the present technique. In Fig. 15, the vertical axis represents a propagation delay time and the horizontal axis represents an amount of moisture. In Fig. 15, a straight line is obtained by determining a difference between a propagation transmission time and a reciprocation delay time for each amount of moisture in Fig. 14.

As indicated in Fig. 15, the propagation delay time increases with the amount of moisture, and thus both are in proportion to each other. Accordingly, Formula 4 is established. The coefficient a in Formula 4 is a slope of the straight line in Fig. 15 and the coefficient b is an intercept.

[Measuring operation example of a calibration factor]

Fig. 16 is a flowchart showing a measuring operation example of the calibration factors (ED, ERX, ES, EX, ET). In this example, as calibration standard data, the electrical connection of the third switching unit 116 is sequentially switched to the first terminal 100S, the second terminal 100O, the third terminal 100L, and the fourth terminal 100T (see Fig. 2) upon shipment, and reflection properties ΓS, ΓO, and ΓL and a transmittance property TT for the respective terminals have been measured.

First, the calibration factor calculation unit 405 outputs a calibration start signal to the controller 370 through the communication unit 410 and the communication unit 360. The controller 370 performs processing in the processing order of Fig. 16.

The first switching unit 112 switches electrical connection to the calibration standard 100 (step S100). Thereafter, the third switching unit 116 switches electrical connection to the first terminal 100S (step S102). Subsequently, the transmitter 320 transmits an incident wave to the calibration standard 100, and the reflection coefficient calculation unit 372 stores the reflection coefficient of the first terminal 100S as a reflection property SllmS in the coefficient storing unit 450.

The third switching unit 116 then switches the electrical connection to the second terminal 100O (step S104). Subsequently, the transmitter 320 transmits an incident wave to the calibration standard 100, and the reflection coefficient calculation unit 372 stores the reflection coefficient of the second terminal 100O as a reflection property SllmO in the coefficient storing unit 450.

The third switching unit 116 then switches the electrical connection to the third terminal 100L (step S106). Subsequently, the transmitter 320 transmits an incident wave to the calibration standard 100, and the reflection coefficient calculation unit 372 stores the reflection coefficient of the third terminal 100L as a reflection property S11mL in the coefficient storing unit 450. The transmission coefficient calculation unit 373 stores the transmission coefficient of the third terminal 100L as a transmission property S21mL in the coefficient storing unit 450.

The third switching unit 116 then switches the electrical connection to the fourth terminal 100T, and the second switching unit 114 switches electrical connection to the calibration standard 100 (step S108). Subsequently, the transmitter 320 transmits an incident wave to the calibration standard 100, and the reflection coefficient calculation unit 372 stores the reflection coefficient of the fourth terminal 100T as a reflection property SllmT in the coefficient storing unit 450. The transmission coefficient calculation unit 373 stores the transmission coefficient of the fourth terminal 100T as a transmission property S21mT in the coefficient storing unit 450.

[Math. 1]

$$\begin{bmatrix} ED \\ ERX \\ ES \end{bmatrix} = \begin{bmatrix} 1 & \varGamma S & \varGamma S \cdot S11mS \\ 1 & \varGamma O & \varGamma O \cdot S11mO \\ 1 & \varGamma L & \varGamma L \cdot S11mL \end{bmatrix}^{-1} \begin{bmatrix} S11mS \\ S11mO \\ S11mL \end{bmatrix}$$

Formula (5)

[Math. 2]

$$EX = S21mL$$

Formula (6)

[Math. 3]

$$S11aT = \frac{S11mT - ED}{ERX + ES \cdot S11mT}$$

Formula (7)

[Math. 4]

$$ET = (ES \cdot S11aT - 1)\frac{EX - S21mT}{TT}$$

Formula (8)

The calibration factor calculation unit 405 calculates the calibration factors (ED, ERX, ES, EX, ET) according to, for example, Formulas (5) to (8) and stores the calibration factors in the coefficient storing unit 450 (step S110).

The calibration factor calculation unit 405 then outputs a calibration termination signal to the controller 370 through the communication unit 410 and the communication unit 360. The first switching unit 112 switches the electrical connection to the probe 201, and the second switching unit 114 switches the electrical connection to the probe 202, which terminates the measuring operation of the calibration factors (step S112).

[Measuring operation example of measurement device]

Fig. 17 is a flowchart showing an operation example of the measurement device 1 according to the first embodiment of the present technique. The operation in Fig. 17 is started, for example, when a predetermined application for measuring an amount of moisture is executed.

The pair of probes 201 and 202 transmit and receive electromagnetic waves (step S200). The measurement unit 300 calculates a reflection coefficient from incident waves and reflected waves (step S202) and calculates a transmission coefficient from incident waves and transmitted waves (step S204).

Thereafter, the signal processing unit 400 calibrates the reflection coefficient (step S206) and calibrates the transmission coefficient (S208). The signal processing unit 400 then calculates a reciprocation delay time from the calibrated reflection coefficient (step S208) and calculates a propagation transmission time from the calibrated transmission coefficient (step S210). Subsequently, the signal processing unit 400 calculates a propagation delay time from the reciprocation delay time and the propagation transmission time (step S212) and calculates the amount of moisture from the propagation delay time and the coefficients a and b (step S214). After step S214, the measurement device 100 ends the operation for measurement.

Fig. 18 illustrates a configuration example of a measurement device 1a as a comparative example. As illustrated in Fig. 18, the calibration standard 100 is not provided in the measurement device 1a. Thus, in the measurement device 1a, a calibration factor is calculated at the factory shipment.

However, the lengths of the cables 308 and 309 are changed by a temperature change or the like. Thus, the calibration factors (ED, ERX, ES, EX, ET) at the shipment may fail to enable proper calibration corresponding to a temperature change or the like, increase an error between a true value and a calibrated value obtained by the calibration factors (ED, ERX, ES, EX, ET) for a measured value, and reduce the measurement accuracy of the amount of moisture. Moreover, in the measurement device 1a, the cables 308 and 309 may vary in shape during the measurement of the calibration standard 100 and the measurement of moisture. This may also increase an error between a true value and a calibrated value obtained by the calibration factors (ED, ERX, ES, EX, ET) for a measured value and reduce the measurement accuracy of the amount of moisture.

However, the measurement device 1 can calculate the calibration factors (ED, ERX, ES, EX, ET) at proper timing before measurement. Thus, even if the lengths of the cables 308 and 309 are changed by a temperature change or the like, the calibration factors (ED, ERX, ES, EX, ET) can be obtained at the time of the change. This can improve the measurement accuracy of the amount of moisture as compared with the case where the calibration factors (ED, ERX, ES, EX, ET) are fixed values. Moreover, in the measurement device 1 according to the present embodiment, the probe 201 and the probe 202 and the calibration standard 100 are fixed at a predetermined positional relationship even during the measurement of moisture, thereby suppressing a change of the shapes of the cables 308 and 309 during the measurement of moisture and the measurement of the calibration factors. This can improve the measurement accuracy of the amount of moisture as compared with the measurement of the calibration factors (ED, ERX, ES, EX, ET) by the removable calibration standard 100.

As described above, according to the present embodiment, the measurement device 1 includes the calibration standard 100 that is fixed at a predetermined positional relationship with the probe 201 and the probe 202 during the measurement of moisture and can be electrically connected to the first connection cable 308 and the second connection cable 309 when moisture is not measured. Thus, even if the lengths of the cables 308 and 309 are changed by a temperature change or the like, the calibration factors (ED, ERX, ES, EX, ET) can be obtained at the time of the change. The probe 201 and the probe 202 and the calibration standard 100 are fixed at a predetermined positional relationship even during the measurement of moisture, thereby suppressing a change of the shapes of the cables 308 and 309 during the measurement of moisture and the measurement of the calibration factors. This can improve the measurement accuracy of the amount of moisture as compared with the case where the calibration factors (ED, ERX, ES, EX, ET) are fixed values.

(Second Embodiment)

The measurement device 1 according to the first embodiment is configured such that the first switching unit 112 and the second switching unit 114 are separated from the calibration standard 100. A measurement device 1 according to a second embodiment is different from the first embodiment in that a first switching unit 112 and a second switching unit 114 are integrated in a calibration standard 100a. Differences from the measurement device 1 according to the first embodiment will be described below.

Fig. 19 illustrates a configuration example of the measurement device 1 according to the second embodiment. As illustrated in Fig. 19, the measurement device 1 according to the second embodiment includes the calibration standard 100a.

Fig. 20 illustrates a configuration example of the calibration standard 100a according to the second embodiment. As illustrated in Fig. 20, the calibration standard 100a according to the second embodiment includes a first switching unit 112a, a second switching unit 114a, and four standards of open, short, load, and through. Furthermore, the calibration standard 100 includes a first terminal 100S for connection to a short-circuit standard, a second terminal 100O for connection to an open standard, a third terminal 100L for connection to a reflection-free termination (load standard), fourth terminals 100Ta and 100Tb for direct connection to a first connection cable 308 and a second connection cable 309, a fifth terminal 100Pa for connection to a first cable 3080, and a sixth terminal 100Pb for connection to a second cable 3090. The first switching unit 112a, the second switching unit 114a, and the four standards are configured thus in the same housing, thereby further suppressing the influence of an external environment including a humidity. Moreover, the need for the configuration of a third switching unit 116 can be eliminated.

Fig. 21 is a flowchart showing a measuring operation example of calibration factors (ED, ERX, ES, EX, ET) according to the second embodiment. In this example, as calibration standard data, the electrical connection of the third switching unit 116 is sequentially switched to the first terminal 100S, the second terminal 100O, the third terminal 100L, and the fourth terminal 100T (see Fig. 2) upon shipment, and reflection properties $\Gamma S$, $\Gamma O$, and $\Gamma L$ and a transmittance property TT for the respective terminals have been measured.

The first switching unit 112a switches the electrical connection to the first terminal 100S (step S302). Subsequently, a transmitter 320 transmits an incident wave to the calibration standard 100a, and a reflection coefficient calculation unit 372 stores the reflection coefficient of the first terminal 100S as a reflection property SllmS in a coefficient storing unit 450.

The first switching unit 112a switches the electrical connection to the second terminal 100O (step S304). Subsequently, the transmitter 320 transmits an incident wave to the calibration standard 100a, and the reflection coefficient calculation unit 372 stores the reflection coefficient of the second terminal 100O as a reflection property SllmT in the coefficient storing unit 450.

The first switching unit 112a then switches the electrical connection to the third terminal 100L (step S306). Subsequently, the transmitter 320 transmits an incident wave to the calibration standard 100a, and the reflection coefficient calculation unit 372 stores the reflection coefficient of the third terminal 100L as a reflection property S11mL in the coefficient storing unit 450. The transmission coefficient calculation unit 373 stores the transmission coefficient of the third terminal 100L as a transmission property S21mL in the coefficient storing unit 450.

The first switching unit 112a then switches the electrical connection to the fourth terminal 100Ta, and the second switching unit 114a switches electrical connection to the fourth terminal 100Tb (step S308). Subsequently, the transmitter 320 transmits an incident wave to the calibration standard 100a, and the reflection coefficient calculation unit 372 stores the reflection coefficient of the fourth terminal 100T as the reflection property SllmT in the coefficient storing unit 450. The transmission coefficient calculation unit 373 stores the transmission coefficient of the fourth terminal 100T as a transmission property S21mT in the coefficient storing unit 450.

A calibration factor calculation unit 405 then calculates the calibration factors (ED, ERX, ES, EX, ET) according to, for example, Formulas (5) to (8) and stores the calibration factors in the coefficient storing unit 450 (step S310).

The calibration factor calculation unit 405 then outputs a calibration termination signal to a controller 370 through a communication unit 410 and a communication unit 360. The first switching unit 112 switches the electrical connection to a probe 201, and the first switching unit 112a and the second switching unit 114a switch the electrical connection to a probe 202, which terminates the measuring operation of the calibration factors (step S312).

As described above, according to the present embodiment, the first switching unit 112 and the second switching unit 114 are configured in the calibration standard 100a. This can further suppress the influence of an external environment including a humidity. Moreover, the need for the configuration of the third switching unit 116 can be eliminated.

(Third Embodiment)

A measurement device 1 according to a third embodiment is different from the measurement device 1 according to the second embodiment in that a calibration standard 100 has a symmetrical configuration. Differences from the measurement device 1 according to the second embodiment will be described below.

Fig. 22 illustrates a configuration example of a calibration standard 100b according to the third embodiment. As illustrated in Fig. 20, the calibration standard 100b according to the second embodiment includes a first switching unit 112b, a second switching unit 114b, and four standards of open, short, load, and through. Furthermore, the calibration standard 100 includes first terminals 100Sa and 100Sb for connection to an open standard, second terminals 100Oa and 100Ob for connection to a short standard, third terminals 100La and 100Lb for connection to a reflection-free termination (load standard), fourth terminals 100Ta and 100Tb for direct connection to a first connection cable 308 and a second connection cable 309, a fifth terminal 100Pa for connection to a first cable 3080, and a sixth terminal 100Pb for connection to a second cable 3090. The calibration standard 100a having the symmetrical configuration can improve the calculation accuracy of calibration factors (ED, ERX, ES, EX, ET). The first switching unit 112a, the second switching unit 114a, and the four standards are configured in the same housing as in the calibration standard 100a according to the second embodiment, thereby further suppressing the influence of an external environment including a humidity.

Fig. 23 is a flowchart showing a measuring operation example of the calibration factors (ED, ERX, ES, EX, ET) according to the third embodiment. In this example, as calibration standard data, the electrical connection of a third switching unit 116 is sequentially switched to the first terminal 100S, the second terminal 100O, the third terminal 100L, and the fourth terminal 100T (see Fig. 2) upon shipment, and reflection properties $\Gamma S$, $\Gamma O$, and $\Gamma L$ and a transmittance property TT for the respective terminals have been measured.

The first switching unit 112b and the second switching unit 114b switch the electrical connection to the first terminals 100Sa and 100Sb (step S302). Subsequently, a transmitter 320 transmits an incident wave to the calibration standard 100b, and a reflection coefficient calculation unit 372 stores the reflection coefficient of the first terminal 100S as a reflection property SllmS in a coefficient storing unit 450.

The first switching unit 112b and the second switching unit 114ba then switch the electrical connection to the second terminals 100Oa and 100Ob (step S404). Subsequently, the transmitter 320 transmits an incident wave to the calibration standard 100b, and the reflection coefficient calculation unit 372 stores the reflection coefficient of the second terminal 100O as a reflection property SllmT in the coefficient storing unit 450.

The first switching unit 112b and the second switching unit 114ba then switch the electrical connection to the third terminals 100La and 100Lb (step S406). Subsequently, the transmitter 320 transmits an incident wave to the calibration standard 100b, and the reflection coefficient calculation unit 372 stores the reflection coefficient of the third terminal 100L as a reflection property S11mL in the coefficient storing unit 450. A transmission coefficient calculation unit 373 stores the transmission coefficient of the third terminal 100L as a transmission property S21mL in the coefficient storing unit 450.

The first switching unit 112b and the second switching unit 114ba then switch the electrical connection to the fourth terminals 100Ta and 100Tb (step S308). Subsequently, the transmitter 320 transmits an incident wave to the calibration standard 100b, and the reflection coefficient calculation unit 372 stores the reflection coefficient of the fourth terminal 100T as the reflection property SllmT in the coefficient storing unit 450. The transmission coefficient calculation unit 373 stores the transmission coefficient of the fourth terminal 100T as a transmission property S21mT in the coefficient storing unit 450.

A calibration factor calculation unit 405 then calculates the calibration factors (ED, ERX, ES, EX, ET) according to, for example, Formulas (5) to (8) and stores the calibration factors in the coefficient storing unit 450 (step S310).

The calibration factor calculation unit 405 then outputs a calibration termination signal to a controller 370 through a communication unit 410 and a communication unit 360. The first switching unit 112b and the second switching unit 114ba switches the electrical connection to probes 201 and 202 and terminates the measuring operation of the calibration factors (step S412).

As described above, according to the present embodiment, the calibration standard 100a is symmetrically configured. Thus, the calculation accuracy of the calibration factors (ED, ERX, ES, EX, ET) can be improved by the symmetrical configuration of the calibration standard 100a.

(Fourth Embodiment)

A measurement device 1 according to a fourth embodiment is different from the solid-state imaging device 1 according to the first embodiment in that a plurality of sensor devices 2 and a signal processing device 4 are provided in different housings. Differences from the solid-state imaging device 1 according to the first embodiment will be described below.

Fig. 24 illustrates a configuration example of the measurement device 1 according to the fourth embodiment. As illustrated in Fig. 24, the measurement device 1 according to the fourth embodiment includes the plurality of sensor devices 2 and the signal processing device 4.

The signal processing device 4 includes a measurement unit 300a and a signal processing unit 400. The measurement unit 300a includes a switching element 3000 that can electrically switch connections to the plurality of sensor devices 2. The switching element 3000 switches an electrical connection for each of the sensor devices 2 under the control of the signal processing unit 400. Thus, calibration factors (ED, ERX, ES, EX, ET) can be calculated and a measuring operation can be performed for each of the sensor devices 2.

As illustrated in Fig. 24, the plurality of sensor devices 2 and the signal processing device 4 are configured in different housings. Thus, the measurement device 1 can be configured with extended cables 308, 309, and 310. This can locate the signal processing device 4 farther than probes 201 and 202. However, as cables 308 and 309 are extended, an error of the calibration factors (ED, ERX, ES, EX, ET) increases with a temperature change. However, the measurement device 1 can properly calculate the calibration factors (ED, ERX, ES, EX, ET) for each of the sensor devices 2. Thus, the measurement accuracy of the amount of moisture can be improved even if the cables 308, 309, and 310 are extended.

As described above, according to the present embodiment, the plurality of sensor devices 2 and the signal processing device 4 are configured in different housings. Thus, the signal processing device 4 can be disposed farther than the probes 201 and 202. Moreover, the calibration factors (ED, ERX, ES, EX, ET) can be properly calculated for each of the sensor devices 2. Thus, the measurement accuracy of the amount of moisture can be improved even if the cables 308, 309, and 310 are extended.

(Fifth Embodiment)

A measurement device 1 according to a fifth embodiment is different from the measurement device 1 according to the fourth embodiment in that radio communications can be carried out. Differences from the measurement device 1 according to the fourth embodiment will be described below.

Fig. 25 illustrates a configuration example of the measurement device 1 according to the fifth embodiment. As illustrated in Fig. 25, the measurement device 1 according to the fourth embodiment includes a plurality of sensor devices 2 and a signal processing device 4.

As illustrated in Fig. 25, the signal processing device 4 is different from the signal processing device 4 according to the fourth embodiment in that an antenna 500 is further provided and a communication unit 410 (see Fig. 10) can perform radio communications. With this configuration, calibration factors (ED, ERX, ES, EX, ET) or the like can be stored for each of the sensor devices 2 in a cloud database 4000. Thus, the calibration factors (ED, ERX, ES, EX, ET) or the like can be managed for each of the sensor devices 2 by the cloud database 4000. This allows the cloud database 4000 to monitor the states of the plurality of sensor devices 2. Furthermore, the storage capacity of a coefficient storing unit 450 (see Fig. 10) can be reduced. The cloud database 4000 may have the functions of a signal processing unit. This can further simplify the configuration of the signal processing device 4.

As described above, according to the present embodiment, the signal processing device 4 is configured to wirelessly communicate with the cloud database 4000. With this configuration, the calibration factors (ED, ERX, ES, EX, ET) or the like can be stored for each of the sensor devices 2 in the cloud database 4000. Thus, the calibration factors (ED, ERX, ES, EX, ET) or the like can be managed for each of the sensor devices 2 by the cloud database 4000.

The present technique can be configured as follows:

(1) A measurement device configured to measure an amount of moisture contained in a medium, the measurement device including:

a first probe in which a first cable electrically connectable to a first connection cable is embedded;
a second probe in which a second cable electrically connectable to a second connection cable is embedded; and
a standard that is fixed at a predetermined positional relationship with the first probe and the second probe even during the measurement, is electrically connectable to the first connection cable and the second connection cable when the measurement is not conducted, and is used for calibration of the measurement.

(2) The measurement device according to (1), further including a switching unit that switches connection between the first connection cable and the first probe or the standard and connection between the second connection cable and the second probe or the standard.

(3) The measurement device according to (2), wherein the switching unit includes:

a first switching unit that switches the connection between the first connection cable and the first probe or the standard; and
a second switching unit that switches the connection between the second connection cable and the second probe or the standard.

(4) The measurement device according to (3), wherein the standard includes an open standard, a short standard, and a reflection-free termination and further includes a first terminal for connection to the open standard, a second terminal for connection to the short standard, a third terminal for connection to the reflection-free termination, and one fourth terminal and the other fourth terminal for direct connection to the first connection cable and the second connection cable.

(5) The measurement device according to (4), wherein the first switching unit switches connection between the first connection cable and one of the first probe, the first to third terminals, and the one fourth terminal, and the second switching unit switches connection between the second connection cable and the second probe or the other fourth terminal.

(6) The measurement device according to (4) or (5), wherein the first probe, the second probe, the standard, the first switching unit, and the second switching unit are configured in the same housing.

(7) The measurement device according to (6), wherein the first switching unit, the first to fourth terminals, and the second switching unit have a symmetrical configuration.

(8) The measurement device according to one of (1) to (7), further including a coefficient calculation unit that determines, as a reflection coefficient, a ratio between complex amplitudes of an incident wave and a reflected wave, the incident wave being transmitted to the first probe through the first cable, the reflected wave being obtained from reflection of the incident wave on the first probe, and

determines, as a transmission coefficient, a ratio between complex amplitudes of the incident wave and a transmitted wave obtained through the medium between the first probe and the second probe;

a calibration unit that calibrates the reflection coefficient and the transmission coefficient according to calibration factors determined by using the standard; and a processing unit that measures an amount of moisture contained in the medium on the basis of the calibrated reflection coefficient and the calibrated transmission coefficient.

(9) The measurement device according to (8), wherein the first probe, the second probe, and the standard are configured as a measurement device in the same housing.

(10) The measurement device according to (9), wherein the coefficient calculation unit, the calibration unit, and the processing unit are configured as a signal processing device in the same housing.

(11) The measurement device according to (10), wherein the measurement device and the signal processing device are integrated in the same housing.

(12) The measurement device according to (10), wherein the measurement device and the signal processing device are configured in separated different housings.

(13) The measurement device according to (10), wherein the plurality of measurement devices and the signal processing device are connected to each other.

(14) The measurement device according to (13), wherein the signal processing device is capable of radio communications.

(15) A measuring method for a measurement device including: a first probe in which a first cable electrically connectable to a first connection cable is embedded;

a second probe in which a second cable electrically connectable to a second connection cable is embedded; and a standard that is fixed at a predetermined positional relationship with the first probe and the second probe even during the measurement, is electrically connectable to the first connection cable and the second connection cable when the measurement is not conducted, and is used for calibration of the measurement,

the method including: a calculating step of transmitting incident waves to the standard through the first connection cable in a predetermined order and calculating calibration factors on the basis of measurement data sequentially measured through the second connection cable;

a coefficient calculating step of determining, as a reflection coefficient, a ratio between complex amplitudes of an incident wave and a reflected wave, the incident wave being transmitted to the first probe through the first cable, the reflected wave being obtained from reflection of the incident wave on the first probe, and

determining, as a transmission coefficient, a ratio between complex amplitudes of the incident wave and a transmitted wave obtained through the medium between the first probe and the second probe;

a calibrating step of calibrating the reflection coefficient and the transmission coefficient according to the calibration factors; and

a processing step of performing processing for measuring an amount of moisture contained in the medium on the basis of the calibrated reflection coefficient and the calibrated transmission coefficient.

Aspects of the present disclosure are not limited to the aforementioned individual embodiments and include various modifications that those skilled in the art can achieve, and effects of the present disclosure are also not limited to the details described above. In other words, various additions, modifications, and partial deletion can be made without departing from the conceptual idea and the gist of the present disclosure that can be derived from the details defined in the claims and the equivalents thereof.

[Reference Signs List]

| | |
|---|---|
| 1 | Measurement device |
| 2 | Sensor device |
| 4 | Signal processing device 4 |
| 201 | Probe |
| 202 | Probe |
| 100, 100a, 100b | Calibration standard |
| 112, 112a, 112b | First switching unit |
| 114, 114a, 114b | Second switching unit |
| 116 | Third switching unit |
| 300 | Measurement unit |

EP 4 283 283 A1

| 308 | First connection cable |
| 309 | Second connection cable |
| 372 | Reflection coefficient calculation unit |
| 373 | Transmission coefficient calculation unit |
| 400 | Signal processing unit |
| 415 | Calibration unit |
| 3080 | First cable |
| 3090 | Second cable |

**Claims**

1. A measurement device configured to measure an amount of moisture contained in a medium, the measurement device comprising:

   a first probe in which a first cable electrically connectable to a first connection cable is embedded;
   a second probe in which a second cable electrically connectable to a second connection cable is embedded; and
   a standard that is fixed at a predetermined positional relationship with the first probe and the second probe even during the measurement, is electrically connectable to the first connection cable and the second connection cable when the measurement is not conducted, and is used for calibration of the measurement.

2. The measurement device according to claim 1, further comprising a switching unit that switches connection between the first connection cable and the first probe or the standard and connection between the second connection cable and the second probe or the standard.

3. The measurement device according to claim 2, wherein the switching unit includes:

   a first switching unit that switches the connection between the first connection cable and the first probe or the standard; and
   a second switching unit that switches the connection between the second connection cable and the second probe or the standard.

4. The measurement device according to claim 3, wherein the standard includes an open standard, a short standard, and a reflection-free termination and further includes a first terminal for connection to the open standard, a second terminal for connection to the short standard, a third terminal for connection to the reflection-free termination, and one fourth terminal and the other fourth terminal for direct connection to the first connection cable and the second connection cable.

5. The measurement device according to claim 4, wherein the first switching unit switches connection between the first connection cable and one of the first probe, the first to third terminals, and the one fourth terminal, and the second switching unit switches connection between the second connection cable and the second probe or the other fourth terminal.

6. The measurement device according to claim 4, wherein the first probe, the second probe, the standard, the first switching unit, and the second switching unit are configured in the same housing.

7. The measurement device according to claim 6, wherein the first switching unit, the first to fourth terminals, and the second switching unit have a symmetrical configuration.

8. The measurement device according to claim 1, further comprising a coefficient calculation unit that determines, as a reflection coefficient, a ratio between complex amplitudes of an incident wave and a reflected wave, the incident wave being transmitted to the first probe through the first cable, the reflected wave being obtained from reflection of the incident wave on the first probe, and determines, as a transmission coefficient, a ratio between complex amplitudes of the incident wave and a transmitted wave obtained through the medium between the first probe and the second probe;

   a calibration unit that calibrates the reflection coefficient and the transmission coefficient according to calibration factors determined by using the standard; and

19

a processing unit that measures an amount of moisture contained in the medium on the basis of the calibrated reflection coefficient and the calibrated transmission coefficient.

9. The measurement device according to claim 8, wherein the first probe, the second probe, and the standard are configured as a sensor device in the same housing.

10. The measurement device according to claim 9, wherein the coefficient calculation unit, the calibration unit, and the processing unit are configured as a signal processing device in the same housing.

11. The measurement device according to claim 10, wherein the sensor device and the signal processing device are integrated in the same housing.

12. The measurement device according to claim 10, wherein the sensor device and the signal processing device are configured in separated different housings.

13. The measurement device according to claim 10, wherein the plurality of sensor devices and the signal processing device are connected to each other.

14. The measurement device according to claim 13, wherein the signal processing device is capable of radio communications.

15. A measuring method for a measurement device, the measurement device including: a first probe in which a first cable electrically connectable to a first connection cable is embedded;

a second probe in which a second cable electrically connectable to a second connection cable is embedded; and a standard that is fixed at a predetermined positional relationship with the first probe and the second probe even during the measurement, is electrically connectable to the first connection cable and the second connection cable when the measurement is not conducted, and is used for calibration of the measurement, the method comprising: a calculating step of transmitting incident waves to the standard through the first connection cable in a predetermined order and calculating calibration factors on a basis of measurement data sequentially measured through the second connection cable;
a coefficient calculating step of determining, as a reflection coefficient, a ratio between complex amplitudes of an incident wave and a reflected wave, the incident wave being transmitted to the first probe through the first cable, the reflected wave being obtained from reflection of the incident wave on the first probe, and
determining, as a transmission coefficient, a ratio between complex amplitudes of the incident wave and a transmitted wave obtained through the medium between the first probe and the second probe;
a calibrating step of calibrating the reflection coefficient and the transmission coefficient according to the calibration factors; and
a processing step of performing processing for measuring an amount of moisture contained in the medium on the basis of the calibrated reflection coefficient and the calibrated transmission coefficient.

Fig. 1

Fig. 2

Fig. 3

Fig. 4

210

240

213 $\varepsilon^*$

$\varepsilon_c$

211 212

3080

$\underline{a}$

511

512

513

$\underline{b}$

Fig. 5

Fig. 6

Fig. 7

Fig. 8

TRANSMITTER

INCIDENT
WAVE
(310) ◄———  321 — 322  — 320

a

330

INCIDENT WAVE RECEIVER

INCIDENT
WAVE
(310) ———  331  — BAND PASS FILTER — 332  333 ADC ——► (370)

LOCAL SIGNAL
(340)

b

(330)  340

REFLECTED WAVE RECEIVER

REFLECTED
WAVE
(310) ———  341  — BAND PASS FILTER — 342  343 ADC ——► (370)

LOCAL SIGNAL
(350)

c

(340)  350

TRANSMITTED WAVE
RECEIVER  354  355

TRANSMITTED
WAVE
(202) ———  351  — LOCAL SIGNAL  353 — BAND PASS FILTER — ADC ——► (370)

309  LOCAL SIGNAL

352

d

28

Fig. 9

(360)

REFLECTION
COEFFICIENT

TRANSMISSION
COEFFICIENT

CONTROLLER                                    370

371

(320) ←—— TRANSMISSION
CONTROLLER

372

(330) ——→ REFLECTION
COEFFICIENT
CALCULATION
UNIT

(340) ——→

373

TRANSMISSION
COEFFICIENT
CALCULATION
UNIT

(350) ——→

374

SWITCHING
UNIT        ——→ (100)

——→ (110)

Fig. 10

SIGNAL PROCESSING UNIT

MEASUREMENT
DATA
(300)

CALIBRATION
PROCESSING
INSTRUCTION
SIGNAL

COMMUNICATION
UNIT ⌇410

CALIBRATION
FACTOR
CALCULATION
UNIT ⌇405

CALIBRATION
UNIT ⌇415

REFLECTION
COEFFICIENT

TRANSMISSION
COEFFICIENT

RECIPROCATING
DELAY TIME
CALCULATION
UNIT ⌇420

PROPAGATION
TRANSMISSION TIME
CALCULATION UNIT ⌇430

MOISTURE
AMOUNT
MEASUREMENT
UNIT ⌇440

COEFFICIENT
STORING
UNIT ⌇450

AMOUNT OF
MOISTURE

Fig. 11

201

REFLECTED
WAVE

INCIDENT
WAVE

308

EW

D

309

TRANSMITTED
WAVE

202

Fig. 12

| | 0.0% |
| ........... | 10.1% |
| —·—·— | 19.7% |
| —··—··— | 32.9% |

Fig. 13

Fig. 14

Fig. 15

Fig. 16

```
                    ┌──────────┐
                    │  START   │
                    └────┬─────┘
                         │
   ┌─────────────────────────────────────────┐
   │    SWITCH TO CALIBRATION STANDARD        │────S100
   └─────────────────────┬───────────────────┘
                         │
   ┌─────────────────────────────────────────┐
   │ SWITCH TO S AND MEASURE REFLECTION       │────S102
   │         PROPERTY S11mS                   │
   └─────────────────────┬───────────────────┘
                         │
   ┌─────────────────────────────────────────┐
   │ SWITCH TO O AND MEASURE REFLECTION       │────S104
   │         PROPERTY S11mO                   │
   └─────────────────────┬───────────────────┘
                         │
   ┌─────────────────────────────────────────┐
   │ SWITCH TO L AND MEASURE REFLECTION       │
   │ PROPERTY S11mL AND TRANSMISSION          │────S106
   │         PROPERTY S21mL                   │
   └─────────────────────┬───────────────────┘
                         │
   ┌─────────────────────────────────────────┐
   │ SWITCH TO T AND MEASURE REFLECTION       │
   │ PROPERTY S11mT AND TRANSMISSION          │────S108
   │         PROPERTY S21mT                   │
   └─────────────────────┬───────────────────┘
                         │
   ┌─────────────────────────────────────────┐
   │   CALCULATE CALIBRATION FACTOR           │────S110
   │     BY USING STANDARD DATA               │
   └─────────────────────┬───────────────────┘
                         │
   ┌─────────────────────────────────────────┐
   │     SWITCH TO ANTENNA SIDE               │────S112
   └─────────────────────┬───────────────────┘
                         │
                    ┌──────────┐
                    │   END    │
                    └──────────┘
```

Fig. 17

```
                    ┌─────────────┐
                    │    START    │
                    └──────┬──────┘
                           │
        ┌──────────────────────────────┐
        │   TRANSMIT AND RECEIVE        │──── S200
        │   ELECTROMAGNETIC WAVE        │
        └──────────────┬───────────────┘
                       │
        ┌──────────────────────────────┐
        │   CALCULATE REFLECTION        │──── S202
        │   COEFFICIENT                 │
        └──────────────┬───────────────┘
                       │
        ┌──────────────────────────────┐
        │   CALCULATE TRANSMISSION      │──── S204
        │   COEFFICIENT                 │
        └──────────────┬───────────────┘
                       │
        ┌──────────────────────────────┐
        │   CALIBRATE REFLECTION        │──── S206
        │   COEFFICIENT                 │
        └──────────────┬───────────────┘
                       │
        ┌──────────────────────────────┐
        │   CALIBRATE TRANSMISSION      │──── S208
        │   COEFFICIENT                 │
        └──────────────┬───────────────┘
                       │
        ┌──────────────────────────────┐
        │   CALCULATE RECIPROCATION     │──── S210
        │   DELAY TIME                  │
        └──────────────┬───────────────┘
                       │
        ┌──────────────────────────────┐
        │   CALCULATE TRANSMISSION      │──── S212
        │   PROPAGATION TIME            │
        └──────────────┬───────────────┘
                       │
        ┌──────────────────────────────┐
        │   CALCULATE TRANSMISSION      │──── S214
        │   DELAY TIME                  │
        └──────────────┬───────────────┘
                       │
        ┌──────────────────────────────┐
        │   CALCULATE AMOUNT OF         │──── S216
        │   MOISTURE                    │
        └──────────────┬───────────────┘
                       │
                ┌─────────────┐
                │     END     │
                └─────────────┘
```

Fig. 18

Fig. 19

SIGNAL
PROCESSING UNIT — 400

4

409

110

308

309

310

MEASUREMENT UNIT — 300

CALIBRATION
STANDARD — 100a

3080

2

3090

D

EW

201    202

200

1

Fig. 20

Fig. 21

```
                    ┌─────────────┐
                    │    START    │
                    └─────────────┘
                           │
    ┌──────────────────────────────────────────┐
    │ SWITCH TO S AND MEASURE REFLECTION        │~~S302
    │         PROPERTY S11mS                     │
    └──────────────────────────────────────────┘
                           │
    ┌──────────────────────────────────────────┐
    │ SWITCH TO O AND MEASURE REFLECTION        │~~S304
    │         PROPERTY S11mO                     │
    └──────────────────────────────────────────┘
                           │
    ┌──────────────────────────────────────────┐
    │ SWITCH TO L AND MEASURE REFLECTION        │~~S306
    │   PROPERTY S11mL AND TRANSMISSION          │
    │         PROPERTY S21mL                     │
    └──────────────────────────────────────────┘
                           │
    ┌──────────────────────────────────────────┐
    │ SWITCH TO T AND MEASURE REFLECTION        │~~S308
    │   PROPERTY S11mT AND TRANSMISSION          │
    │         PROPERTY S21mT                     │
    └──────────────────────────────────────────┘
                           │
    ┌──────────────────────────────────────────┐
    │    CALCULATE CALIBRATION FACTOR            │~~S310
    │      BY USING STANDARD DATA                │
    └──────────────────────────────────────────┘
                           │
    ┌──────────────────────────────────────────┐
    │        SWITCH TO ANTENNA SIDE             │~~S312
    └──────────────────────────────────────────┘
                           │
                    ┌─────────────┐
                    │     END     │
                    └─────────────┘
```

Fig. 22

Fig. 23

```
                        ┌──────────┐
                        │  START   │
                        └────┬─────┘
                             │
       ┌─────────────────────┴──────────────────────┐
       │ SWITCH TO S AND MEASURE REFLECTION          │⟋⟍ S402
       │         PROPERTY S11mS                      │
       └─────────────────────┬──────────────────────┘
                             │
       ┌─────────────────────┴──────────────────────┐
       │ SWITCH TO O AND MEASURE REFLECTION          │⟋⟍ S404
       │         PROPERTY S11mO                      │
       └─────────────────────┬──────────────────────┘
                             │
       ┌─────────────────────┴──────────────────────┐
       │ SWITCH TO L AND MEASURE REFLECTION          │⟋⟍ S206
       │  PROPERTY S11mL AND TRANSMISSION            │
       │         PROPERTY S21mL                      │
       └─────────────────────┬──────────────────────┘
                             │
       ┌─────────────────────┴──────────────────────┐
       │ SWITCH TO T AND MEASURE REFLECTION          │⟋⟍ S408
       │  PROPERTY S11mT AND TRANSMISSION            │
       │         PROPERTY S21mT                      │
       └─────────────────────┬──────────────────────┘
                             │
       ┌─────────────────────┴──────────────────────┐
       │     CALCULATE CALIBRATION FACTOR            │⟋⟍ S410
       │       BY USING STANDARD DATA                │
       └─────────────────────┬──────────────────────┘
                             │
       ┌─────────────────────┴──────────────────────┐
       │        SWITCH TO ANTENNA SIDE               │⟋⟍ S412
       └─────────────────────┬──────────────────────┘
                             │
                        ┌────┴─────┐
                        │   END    │
                        └──────────┘
```

Fig. 24

Fig. 25

## INTERNATIONAL SEARCH REPORT

| International application No. |
| --- |
| **PCT/JP2021/046431** |

**A. CLASSIFICATION OF SUBJECT MATTER**

*G01N 22/04*(2006.01)i; *G01N 22/00*(2006.01)i
FI:   G01N22/04 C; G01N22/00 F

According to International Patent Classification (IPC) or to both national classification and IPC

**B. FIELDS SEARCHED**

Minimum documentation searched (classification system followed by classification symbols)

G01N22/00 - G01N22/04, G01V3/12

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2020-187120 A (SONY CORP) 19 November 2020 (2020-11-19) paragraphs [0026]-[0127], fig. 1-20 | 1-15 |
| Y | US 5548538 A (WILTRON COMPANY) 20 August 1996 (1996-08-20) abstract, columns 4-12, fig. 2-6 | 1-15 |
| A | WO 2018/221051 A1 (SONY CORP) 06 December 2018 (2018-12-06) paragraphs [0029]-[0120], fig. 1-22 | 1-15 |
| A | US 2018/0224382 A1 (VAYYAR IMAGING LTD) 09 August 2018 (2018-08-09) paragraphs [0084]-[0177], fig. 1-7B | 1-15 |
| A | EP 3605152 A1 (LOMBARDI SA INGEGNERI CONSULENTI) 05 February 2020 (2020-02-05) paragraphs [0056]-[0088], fig. 1-3 | 1-15 |
| A | KR 10-2019-0066337 A (ISSALAB CO., LTD.) 13 June 2019 (2019-06-13) paragraphs [0001]-[0005], fig. 1 | 1-15 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **26 January 2022** | **08 March 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2021/046431**

| Patent document cited in search report | | | Publication date (day/month/year) | Patent family member(s) | | | Publication date (day/month/year) |
|---|---|---|---|---|---|---|---|
| JP | 2020-187120 | A | 19 November 2020 | WO | 2020/230702 | A1 | |
| | | | | paragraphs [0026]-[0127], fig. 1-20 | | | |
| | | | | WO | 2020/230478 | A1 | |
| | | | | CN | 113795750 | A | |
| US | 5548538 | A | 20 August 1996 | US | 5715183 | A | |
| WO | 2018/221051 | A1 | 06 December 2018 | US | 2020/0182906 | A1 | |
| | | | | paragraphs [0075]-[0176], fig. 1-22 | | | |
| | | | | CN | 110678741 | A | |
| US | 2018/0224382 | A1 | 09 August 2018 | WO | 2017/021950 | A2 | |
| EP | 3605152 | A1 | 05 February 2020 | (Family: none) | | | |
| KR | 10-2019-0066337 | A | 13 June 2019 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018221051 A

- JP H08300197 B